# FASCICULE DE BREVET EUROPEEN

(11) **EP 2 268 743 B1**
(45) Date de publication et mention de la délivrance du brevet: **22.04.2015**
(21) Numéro de dépôt: 09724145.9
(22) Date de dépôt: 24.03.2009
(51) Int. Cl.: C08L 83/04, C09D 183/04, C09J 183/04, C07C 279/04, C07C 279/16, C08K 5/31

(54) **COMPOSES A STRUCTURE GUANIDINE ET LEURS UTILISATIONS COMME CATALYSEURS DE POLYCONDENSATION D'ORGANOPOLYSILOXANES**
VERBINDUNGEN MIT EINER GUANIDINSTRUKTUR UND IHRE VERWENDUNG ALS ORGANOPOLYSILOXAN-POLYKONDENSATIONSKATALYSATOREN
COMPOUNDS HAVING A GUANIDINE STRUCTURE AND USE OF SAME AS ORGANOPOLYSILOXANE POLYCONDENSATION CATALYSTS

(30) Priorité: 28.03.2008 FR 0801707
(43) Date de publication de la demande: 05.01.2011
(73) Titulaire: Bluestar Silicones France, 69486 Lyon Cedex 03 (FR)
(72) Inventeur: BARRANDON, Georges, F-69440 Mornant (FR); BLANC, Delphine, F-69007 Lyon (FR); MALIVERNEY, Christian, F-69690 Saint Julien sur Bibost (FR); PARISOT, Hervé, F-69210 Lentilly (FR)
(74) Mandataire: Mekki, Boualem
(86) Numéro de dépôt international: PCT/EP2009/053425
(87) Numéro de publication internationale: WO 2009/118307

(56) Documents cités:
- WO-A1-2007/094276
- US-A- 4 528 353
- US-A- 5 371 164
- US-A1- 2005 014 894
- DATABASE COMPENDEX [Online] ENGINEERING INFORMATION, INC., NEW YORK, NY, US; 20 juillet 2005 (2005-07-20), KATRITZKY A R; KHASHAB N M; BOBROV S: "The preparation of 1,2,3-trisubstituted guanidines" XP002547260 Database accession no. E2005329291522
- WEN-XIONG ZHANG ET AL: "Catalytic Addition of Amine NH Bonds to Carbodiimides by Half-Sandwich Rare-Earth Metal Complexes: Efficient Synthesis of Substituted Guanidines through Amine Protonolysis of Rare-Earth Metal Guanidinates", CHEMISTRY - A EUROPEAN JOURNAL, vol. 13, no. 14, 7 May 2007 (2007-05-07), pages 4037-4051, XP055142866, ISSN: 0947-6539, DOI: 10.1002/chem.200601383

## Description

La présente invention concerne une composition organopolysiloxanique vulcanisable dès la température ambiante en élastomère réticulant par polycondensation et ne contenant pas de catalyseurs à base d'alkylétain qui présentent des problèmes de toxicité.

L'invention concerne aussi de nouveaux catalyseurs de polycondensation à structure guanidine dans la chimie des silicones et leurs utilisations comme catalyseurs de la réaction de polycondensation d'organopolysiloxanes.

Les formulations des élastomères réticulant par polycondensation font généralement intervenir une huile silicone, généralement un polydiméthylsiloxane, à terminaisons hydroxylées, éventuellement pré-fonctionnalisées par un silane de façon à présenter des extrémités alcoxy, un réticulant, un catalyseur de polycondensation, classiquement un sel d'étain ou un titanate d'alkyle, une charge de renfort et d'éventuels autres additifs comme des charges de bourrage, des promoteurs d'adhérence, des colorants, des agents biocides, etc.

Ces compositions organopolysiloxaniques vulcanisables dès la température ambiante sont bien connues et sont classées en 2 groupes distincts: les compositions monocomposantes (RTV-1) et les compositions bicomposantes (RTV-2).

Le terme "RTV" est l'acronyme pour "Room Temperature Vulcanising".

Lors de la réticulation, l'eau (soit apportée par une humidité atmosphérique dans le cas des RTV-1, soit introduite dans une partie de la composition dans le cas des RTV-2) permet la réaction de polycondensation, qui conduit à la formation du réseau élastomérique.

Généralement, les compositions monocomposantes (RTV-1) réticulent quand elles sont exposées à l'humidité de l'air, c'est-à-dire qu'elles ne peuvent réticuler dans un milieu en confiné. Par exemple, les compositions silicones monocomposantes utilisées comme mastics ou adhésifs réticulent à froid suivant un mécanisme d'hydrolyse de fonctions réactives du type acétoxysilane, cétiminoxysilane, alcoxysilane..., suivi par des réactions de condensation entre des groupements silanols formés et d'autres fonctions réactives résiduelles. L'hydrolyse est généralement effectuée grâce à la vapeur d'eau qui diffuse dans le matériau à partir de la surface exposée à l'atmosphère. Généralement, la cinétique des réactions de polycondensation est extrêmement lente; ces réactions sont donc catalysées par un catalyseur approprié. Comme catalyseurs utilisés, on fait appel le plus souvent à des catalyseurs à base d'étain, de titane, d'une amine ou des compositions de ces catalyseurs. Les catalyseurs à base d'étain (cf. notamment FR-A-2 557 582) et de titane (cf. notamment FR-A-2 786 497) sont des catalyseurs ayant une bonne efficacité. Les élastomères silicones monocomposants à extrémités -Si(OR) sont parfois désignés sous l'appellation élastomères alcoxy.

Quand aux compositions bicomposantes, elles sont commercialisées et stockées sous la forme de deux composantes, une première composante contenant les matériaux polymériques de base et la deuxième composante contenant le catalyseur. Les deux composantes sont mélangées lors de l'emploi et le mélange réticule sous la forme d'un élastomère relativement dur. Ces compositions à deux composantes sont bien connues et sont notamment décrites dans l'ouvrage de Walter Noll "Chemistry and Technology of Silicones" 1968, 2ème édition aux pages 395 à 398. Ces compositions comportent essentiellement 4 ingrédients différents:
- un polymère réactif α,ω-dihydroxydiorganopolysiloxane,
- un agent de réticulation, généralement un silicate ou un polysilicate,
- un catalyseur à l'étain, et
- de l'eau.

Le plus souvent, le catalyseur de condensation est à base d'un composé organique d'étain. En effet, de nombreux catalyseurs à base d'étain ont déjà été proposés comme catalyseur de réticulation de ces RTV-1 ou RTV-2. Des catalyseurs de polycondensation classiques comprennent des composés de dialkylétain, notamment des dicarboxylates de dialkylétain tels que le dilaurate et le diacétate de dibutylétain, les composés de titanate d'alkyle tels que le tétrabutyl ou le tétraisopropyltitanate, les chélates de titane (EP-A-0 885 933, US-5 519 104, US-A-4,515,932, US-A-4,563,498, US-A-4,528,353).

Cependant, les catalyseurs à base d'alkylétain, bien que très efficaces, le plus souvent incolores, liquides et solubles dans les huiles silicones présentent l'inconvénient d'être toxiques (CMR2 toxiques pour la reproduction).

Ainsi, la demande internationale WO 2004/020525 décrit des compositions silicones monocomposantes (RTV-1) utilisées comme mastics ou adhésifs et qui réticulent à froid quand elles sont exposées à l'humidité de l'air et comprenant en plus des composants habituels :
- un réticulant (D) spécifique et essentiel qui est un silane à fonctions 1-méthylvinyloxy connu pour sa forte réactivité comparée à celle des réticulants classiques, et
- des catalyseurs qui sont des dérivés organiques à fonction imines de formules **(I)** ou **(II)** suivantes: 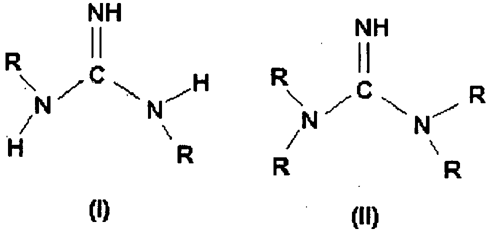
avec R étant un radical spécifique choisi parmi les groupes suivants : méthyle, isopropyle, phényle et orthotolyle. Des exemples de ces dérivés organiques de type imine sont la 1,3-diphénylguanidine, la 1,3-di-o-tolylguanidine, la 1,3-diméthylguanidine et la 1,1,3,3-tétraméthylguanidine qui est le dérivé préféré. Ces dérivés présentent la particularité de posséder une fonction imine non substituée, c'est-à-dire une fonction du type C=NH.

Il est à noter qu'un réticulant classique de type trialcoxysilane, composant (E), est toujours utilisé en association avec le réticulant (D) qui est un silane connu pour sa forte réactivité due à la présence de fonctions de type 1-méthylvinyloxy.

Cependant, le problème associé à l'utilisation des catalyseurs organiques à fonction imines décrits dans la demande internationale WO 2004/020525 est qu'ils doivent être utilisés en présence de réticulants spécifiques très réactif et coûteux (silanes à fonctions 1-méthylvinyloxy), c'est à dire que des réticulants classiques de structures simples, qui sont très largement utilisés dans les formulations RTV-I ou RTV-II, tels que par exemple les alkyltrialcoxysilanes, les silicates d'alkyle ou les polysilicates d'alkyle, ne peuvent leur être associés sans la présence obligatoire d'un réticulant très réactif tel que le silane à fonctions 1-méthylvinyloxy. En effet, sans la présence de ce silane très réactif la réticulation de la composition en élastomère est alors insuffisante et ne permet pas d'obtenir de bonnes propriétés mécaniques. Ainsi, le dérivé 1,1,3,3-tétraméthylguanidine, qui est présenté dans le mode de réalisation préférentiel de cette demande de brevet, lorsqu'il est utilisé avec un réticulant classique, tel que par exemple un polysilicate d'alkyle, et sans la présence d'un silane réactif à fonction méthylvinyloxy, dans un RTV monocomposant (RTV-I), la réticulation, du système est alors insuffisante et ne peut générer un élastomère silicone.

Ces problèmes de réactivité du réticulant par exemple dans les compositions silicones monocomposantes (RTV-1) sont bien connus par l'homme de l'art. En effet, les réticulants alcoxysilanes les plus utilisés sont ceux présentant des groupes méthoxy pour leurs réactivités intrinsèques. Cependant, un des problèmes associés à l'utilisation de ce type d'alcoxysilanes est un dégagement de méthanol source de problèmes sur un plan d'hygiène et de sécurité.

Pour un développement durable, il apparaît donc nécessaire de développer d'autres catalyseurs, non toxiques, pour la réaction de polycondensation des organopolysiloxanes et utilisables à la fois dans la réticulation des compositions élastomères mono- et bi-composantes. De plus, ces catalyseurs doivent être utilisables quel que soit le type de réticulant utilisé.

Un autre aspect important pour un catalyseur de la réaction de polycondensation des organopolysiloxanes est le temps de mise en oeuvre (« pot-life » ou « temps de travail »), c'est-à-dire le temps pendant lequel la composition peut être utilisée après mélange sans durcir. Ce temps doit être suffisamment long, pour permettre son utilisation mais suffisamment court pour obtenir un objet moulé manipulable au plus tard quelques minutes ou quelques heures après sa fabrication. Le catalyseur doit donc permettre d'obtenir un bon compromis entre le temps d'utilisation du mélange catalysé et le temps au bout duquel l'objet moulé est manipulable (ces temps dépendent de l'application visée comme par exemple le moulage ou la fabrication de joints). En outre le catalyseur doit conférer au mélange catalysé un temps d'étalement qui ne varie pas en fonction de la durée au stockage.

Il apparaît aussi nécessaire de fournir une composition organopolysiloxanique à deux composantes (RTV-II) qui possèdent en même temps les propriétés suivantes:
1) une vitesse de prise rapide à température ambiante (en surface et en confiné) tout en conservant un temps de travail suffisamment long (de l'ordre de quelques minutes) pour permettre son utilisation,
2) de bonnes propriétés mécaniques après réticulation,
3) une bonne extrudabilité, et
4) une bonne tenue à la "réversion" de l'élastomère issu de la réticulation de la composition. En effet, lorsque ces élastomères sont soumis à un chauffage immédiatement ou peu après leur réticulation, il se produit très souvent un phénomène que les spécialistes désignent sous le nom de "réversion". Au cours de ce chauffage les élastomères se liquéfient en particulier à coeur. Cette "réversion" peut déjà se produire à des températures supérieures à 80°C. Toutefois, dans la plupart des cas, elle se produit à des températures supérieures à 100°C, et elle est particulièrement marquée lorsque le chauffage des élastomères s'effectue en l'absence totale ou presque de l'air, c'est-à-dire lorsque, au cours du chauffage, les élastomères se trouvent dans un système totalement confiné. Cette réversion se traduit d'abord dans une première phase par une perte de la dureté Shore A, puis lorsque le traitement thermique est maintenu, elle est suivie par une deuxième phase où l'on observe une augmentation à nouveau de la dureté Shore A du fait d'une réticulation résiduelle des fragments générés lors la première phase. Cette "reversion" constitue donc un inconvénient très gênant en particulier pour certaines applications où les élastomères durcis sont chauffés après réticulation ou sont amenés à subir des contraintes thermiques peu après leur réticulation de part l'application visée.

Ainsi, un des objectifs essentiels de la présente invention est de proposer un catalyseur non toxique mais qui continue à répondre à la fois aux contraintes de conservation, de mise en oeuvre et de réticulation des deux types de compositions élastomères mono- et bi-composantes tout en étant utilisable quel que soit le type de réticulant utilisé.

Un autre objectif essentiel de la présente invention est donc de proposer un nouveau catalyseur non toxique permettant aussi bien, à l'humidité de l'air, une réticulation en surface mais aussi une réticulation à coeur la plus complète possible.

Un autre objectif essentiel de la présente invention est de fournir une composition organopolysiloxanique monocomposante (RTV-I) et à deux composantes (RTV-II) comprenant un catalyseur selon l'invention et répondant aux critères énoncés ci-dessus et notamment permettant de préparer des élastomères ayant une bonne tenue à la réversion.

Ces objectifs, parmi d'autres, sont atteints par la présente invention qui concerne tout d'abord une composition organopolysiloxanique ne contenant pas de catalyseur métallique et caractérisée en ce qu'elle comprend d'une part une base silicone **B** comprenant au moins une huile polyorganosiloxane réticulable par réaction de polycondensation de manière à former un élastomère silicone et d'autre part, une quantité catalytiquement efficace d'au moins un catalyseur de polycondensation **A** qui est un composé organique non silylé et répondant à la formule générale **(I):** 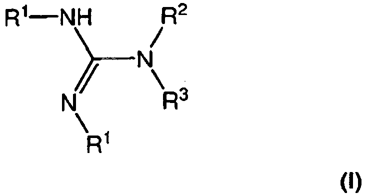 dans laquelle,
- les radicaux R¹, identiques ou différents, représentent, indépendamment l'un de l'autre, un groupe alkyle monovalent linéaire ou ramifié, un groupement cycloalkyle, un groupe (cycloalkyl)alkyle, le cycle étant substitué ou non et pouvant comprendre au moins un hétéroatome ou un groupement fluoroalkyle,
- le radical R² représente un atome d'hydrogène, un groupement alkyle monovalent linéaire ou ramifié, un groupement cycloalkyle, un groupement alkyle substitué par un cycle, substitué ou non et pouvant comprendre au moins un hétéroatome, un groupement aromatique un groupe arylalkyle, un groupement fluoroalkyle, un groupement alkylamine ou alkylguanidine, et
- le radical R³ représente un groupement alkyle monovalent linéaire ou ramifié, un groupement cycloalkyle, un groupement alkyle substitué par un cycle, substitué ou non et pouvant comprendre au moins un hétéroatome, un groupement arylalkyle, fluoroalkyle, alkylamine ou alkylguanidine,
- lorsque le radical R² n'est pas un atome d'hydrogène, les radicaux R² et R³ peuvent être liés pour former un cycle aliphatique à 3, 4, 5, 6 ou 7 chaînons éventuellement substitué par un ou plusieurs substituants, et
- avec la condition supplémentaire que les radicaux R¹, R² et R³ ne comprennent pas d'atome de silicium.

Pour atteindre cet objectif, la Demanderesse a eu le mérite de mettre en évidence, de manière tout à fait surprenante et inattendue, que les composés non silylé et répondant à la formule générale **(I)** permettent de catalyser la réaction de polycondensation des organopolysiloxanes et sont utilisables à la fois dans la réticulation des compositions élastomères mono- et bi-composantes et ceci quel que soit le type de réticulant utilisé.

Il est aussi du mérite des inventeurs d'avoir vaincu le préjugé technique, tel qu'enseigné par exemple par la demande internationale WO 2004/020525, qui voulait que, jusqu'alors, les catalyseurs proches en terme de structure, tels que la 1,3-diphénylguanidine, la 1,3-di-o-tolylguanidine, la 1,3-diméthylguanidine ou la 1,1,3,3-tétraméthylguanidine devaient être associés à des réticulants spécifiques très réactif et coûteux (silanes à fonctions 1-méthylvinyloxy), pour réticuler des formulations de type RTV.

Les composés non silylés selon l'invention et répondant à la formule générale **(I)** sont des guanidines 1,2,3-trisubstituées et 1,2,3,3-tétrasubstituées et présentent l'avantage d'être liquides, incolores, inodores et solubles dans les matrices silicones. Les guanidines non silylées selon l'invention sont mises en oeuvre dans les systèmes silicones à réticuler à des teneurs très faibles, et permettent suivant la teneur d'adapter les temps de travail à l'application tout en garantissant des excellentes duretés des élastomères obtenus, ainsi qu'une excellente stabilité thermique éliminant ainsi les problèmes liés aux phénomènes de réversion.

Selon un mode de réalisation préféré, que le catalyseur de polycondensation **A** est un composé organique non silylé répondant à la formule générale **(I)** et dans laquelle:
- les radicaux R₁, identiques ou différents, et le radical R₃ sont choisis, indépendamment les uns des autres, dans le groupe constitué par: un radical isopropyle, un radical cyclohexyle et un radical alkyle monovalent linéaire ou ramifié en C₁ - C₁₂.
- le radical R₂ représente un atome d'hydrogène, un groupe alkyle monovalent linéaire ou ramifié, un groupe cycloalkyle, un groupe alkyle substitué par un cycle, substitué ou non et pouvant comprendre au moins un hétéroatome, un groupe arylalkyle, un groupe fluoroalkyle, un groupe alkylamine ou alkylguanidine, et
- lorsque le radical R² n'est pas un atome d'hydrogène, les radicaux R² et R³ peuvent être liés pour former un cycle aliphatique à 3, 4, 5, 6 ou 7 chaînons éventuellement substitué par un ou plusieurs substituants.

Des catalyseurs de polycondensation **A** particulièrement préférés sont des composés organiques non silylés choisis parmi le groupe constitué par les composés **(A1)** à **(A6)** suivants:

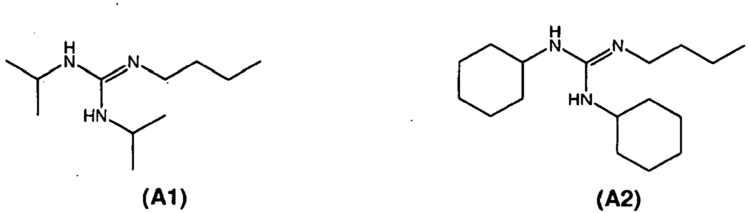

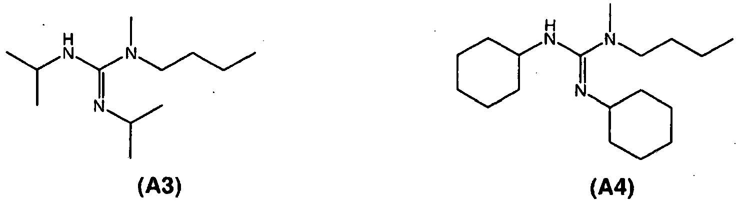

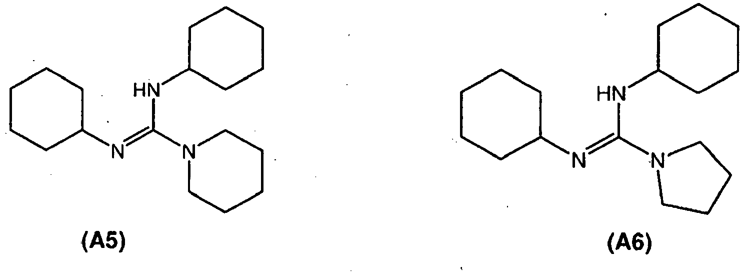

La quantité de catalyseurs de polycondensation A selon l'invention est comprise entre 0,1 et 10 % en poids de la masse totale, de préférence entre 0,1 et 5 %, que ce soit une préparation mono ou bicomposante.

Selon un autre mode de réalisation préféré, la composition selon l'invention telle que décrite ci-dessus est caractérisée en ce qu'elle comprend outre une quantité catalytiquement efficace d'au moins un catalyseur de polycondensation **A** tel que défini ci-dessus et une base silicone **B** comprenant:
- au moins une huile polyorganosiloxane **C** susceptible de réticuler par polycondensation en un élastomère;
- éventuellement au moins un agent de réticulation **D;**
- éventuellement au moins un promoteur d'adhérence **E;** et
- éventuellement au moins une charge minérale siliceuse, organique et/ou non siliceuse **F.**

Pour la réalisation de l'invention, la base silicone **B** peut comprendre :
- pour 100 parties en poids d'au moins une huile polyorganosiloxane **C** susceptible de réticuler par polycondensation qui est un polymère réactif α,ω-dihydroxydiorganopolysiloxane dont les radicaux organiques sont des radicaux hydrocarbonés de préférence choisi parmi le groupe constitué par: les alkyles ayant de 1 à 20 atomes de carbone ; les cycloalkyles ayant de 3 à 8 atomes de carbone ; les alcényles ayant de 2 à 8 atomes de carbone et les cycloalcényles ayant de 5 à 8 atomes de carbone,

- de 0,1 à 60 parties en poids d'au moins un agent de réticulation **D** choisi parmi le groupe constitué par: les polyacoxysilanes, les produits provenant de l'hydrolyse partielle d'un polyalcoxysilane et les polyalcoxysiloxanes,
- de 0 à 60 parties en poids d'un promoteur d'adhérence **E** tel que décrit ci-dessus,
- de 0 à 250 parties en poids, de préférence de 5 à 200 parties en poids, d'au moins une charge minérale siliceuse, organique et/ou non siliceuse **F,**
- de 0,001 à 10 parties en poids d'eau,
- de 0 à 100 parties en poids d'au moins un polymère polyorganosiloxane linéaire non réactif **G** consistant dans un homopolymère ou copolymère linéaire dont, par molécule, les substituants organiques monovalents, identiques ou différents entre eux, liés aux atomes de silicium sont choisis parmi les radicaux alkyles, cycoalkyles, alcényles, aryles, alkylarylènes et arylalkylènes,
- de 0 à 20 parties en poids d'une base colorante ou d'un agent de coloration **H,**
- de 0 à 70 parties en poids de résines polyorganosiloxanes **I,** et
- de 0 à 20 parties d'additifs auxiliaires **J** connus de l'homme de métier, tels que des agents plastifiant, des ralentisseurs de réticulation, des huiles minérales, des agents antimicrobiens, des additifs de tenue thermique tels que les oxydes de titane, de fer ou de cérium.

A ladite base silicone **B** est ajoutée de 0,1 à 50 parties en poids d'au moins un catalyseur de polycondensation **A** selon l'invention et tel que décrit ci dessus.

L'invention concerne aussi un système bicomposant précurseur de la composition organopolysiloxanique selon l'invention et telle que décrite ci-dessus, ladite composition étant vulcanisable en élastomère silicone par des réactions de polycondensation et est caractérisé en ce qu'il se présente en deux parties **P1** et **P2** distinctes destinées à être mélangées pour former ladite composition, et en ce que l'une de ces parties comprend le catalyseur de polycondensation **A** selon l'invention et tel que défini ci-dessus comme catalyseur de la réaction de polycondensation d'organopolysiloxanes et l'agent de réticulation **D** alors que l'autre partie est exempte des espèces précitées et comprend:
- pour 100 parties en poids de l'huile (ou des huiles) polyorganosiloxane(s) **C** susceptible(s) de réticuler par polycondensation en un élastomère, et
- de 0.001 à 10 partie(s) en poids d'eau.

Un autre objet de l'invention consiste en une composition polyorganosiloxanique monocomposante stable au stockage en l'absence d'humidité et réticulant, en présence d'eau, en élastomère, caractérisée en ce qu'elle comprend:
- au moins une huile polyorganosiloxane **C** linéaire réticulable présentant des extrémités fonctionnalisées de type alcoxy, oxime, acyle et /ou énoxy, de préférence alcoxy,
- au moins un agent de réticulation **D,**
- au moins une charge **F,** et
- au moins un catalyseur de la réaction de polycondensation qui est le catalyseur de polycondensation **A** selon l'invention et tel que défini ci-dessus.

Selon un mode de réalisation préféré, la composition polyorganosiloxanique monocomposante décrite ci-dessus est caractérisé en ce que l'huile polyorganosiloxane **C** linéaire réticulable présentant des extrémités fonctionnalisées de type alcoxy est préparée in situ par réaction, en présence d'une quantité catalytiquement efficace de lithine, d'un diorganopolysiloxane linéaire, comprenant un groupement hydroxy lié à un atome de silicium à chaque extrémité de chaîne, avec au moins un polyalcoxysilane de formule **(II)** suivante:

(R⁴)_{c} (R⁵)ₐ Si (OR⁶)_{4-(a+c)} **(II)**

dans laquelle:
- a est égal à 0, 1 ou 2,
- c est égal à 0, 1 ou 2,
- la somme a + c est égale à 0, 1 ou 2,
- R⁴ représente un radical monovalent hydrocarboné saturé ou non en C₁ à C₁₃, substitué ou non, aliphatique, cyclanique ou aromatique, R⁴ pouvant être identique à R⁵,
- R⁵ représente un radical monovalent hydrocarboné saturé ou non en C₁ à C_{13'}, substitué ou non substitué, aliphatique, cyclanique ou aromatique, et pouvant comporter une fonction époxy, amine primaire, secondaire, tertiaire, mercapto, et
- R⁶ représente un radical organique aliphatique ayant de 1 à 8 atomes de carbone choisi notamment entre les radicaux alkyle, les radicaux alkyléther, les radicaux alkylester, les radicaux alkylcétone, les radicaux alkylcyano et les radicaux aralkyle ayant de 7 à 13 atomes de carbone, étant entendu que les groupements alcoxy du silane de formule **(II)** peuvent avoir chacun une signification différente pour R⁶ ou la même signification.

Selon un mode de réalisation préféré, il est avantageux que l'agent de réticulation **D** ou le polyalcoxysilane de formule **(II)** sont choisis parmi le groupe constitué par:
- le vinyltriéthoxysilane,
- le méthyltriéthoxysilane,
- le propyltriéthoxysilane,
- le 1,2-bis(triéthoxysilyl)éthane,
- C₂H₅Si(OC₂H₅)₃, et
- Si(OC₂H₅)₄.

En effet, avec ce type de réticulant, il n'y a plus de dégagement de méthanol comme avec le réticulant classique triméthoxysilane résolvant ainsi de nombreux problèmes d'hygiène et de sécurité.

Selon un autre de ses aspects, la présente invention a également pour objet un élastomère obtenu par réticulation et durcissement du système bi-composant selon l'invention tel que défini ci-dessus, de la composition polyorganosiloxanique monocomposante selon l'invention telle que définie ci-dessus ou de la composition selon l'invention telle que définie ci-dessus.

Un autre objet de l'invention consiste en des composés nouveaux de formules: 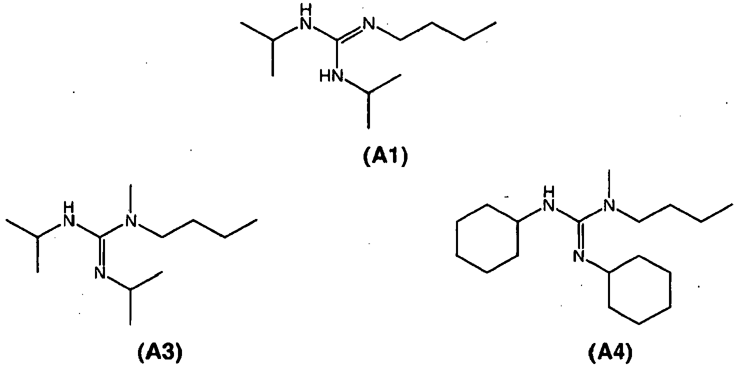 Un autre objet de l'invention consiste en l'utilisation pour catalyser la réaction de polycondensation d'organopolysiloxanes un catalyseur de polycondensation **A** qui est un composé organique non silylé et répondant à la formule générale **(I):** 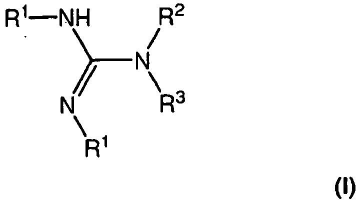 dans laquelle,
- les radicaux R¹, identiques ou différents, représentent, indépendamment l'un de l'autre, un groupe alkyle monovalent linéaire ou ramifié, un groupement cycloalkyle, un groupe (cycloalkyl)alkyle, le cycle étant substitué ou non et pouvant comprendre au moins un hétéroatome ou un groupement fluoroalkyle,
- le radical R² représente un atome d'hydrogène, un groupement alkyle monovalent linéaire ou ramifié, un groupement cycloalkyle, un groupement alkyle substitué par un cycle, substitué ou non et pouvant comprendre au moins un hétéroatome, un groupement aromatique un groupe arylalkyle, un groupement fluoroalkyle, un groupement alkylamine ou alkylguanidine, et
- le radical R³ représente un groupement alkyle monovalent linéaire ou ramifié, un groupement cycloalkyle, un groupement alkyle substitué par un cycle, substitué ou non et pouvant comprendre au moins un hétéroatome, un groupement arylalkyle, fluoroalkyle, alkylamine ou alkylguanidine,
- lorsque le radical R² n'est pas un atome d'hydrogène, les radicaux R² et R³ peuvent être liés pour former un cycle aliphatique à 3, 4, 5, 6 ou 7 chaînons éventuellement substitué par un ou plusieurs substituants, et
- avec la condition supplémentaire que les radicaux R¹, R² et R³ ne comprennent pas d'atome de silicium.

Selon un mode de réalisation préféré, l'utilisation selon l'invention pour catalyser la réaction de polycondensation d'organopolysiloxanes se fait à l'aide d'un catalyseur de polycondensation **A** qui est un composé organique non silylé choisi parmi le groupe constitué par les composés **(A1)** à **(A6)** suivants:

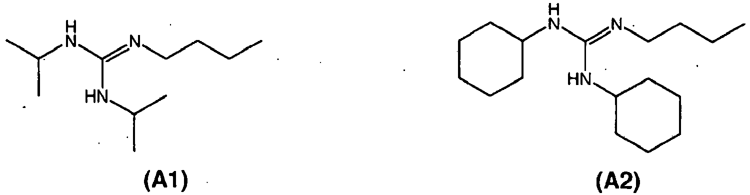

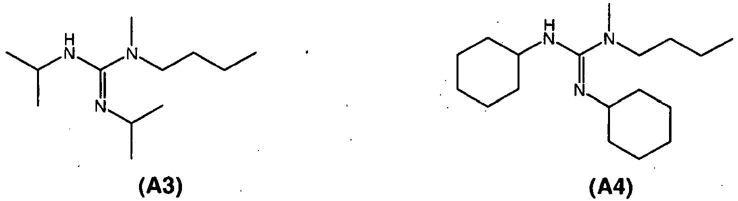

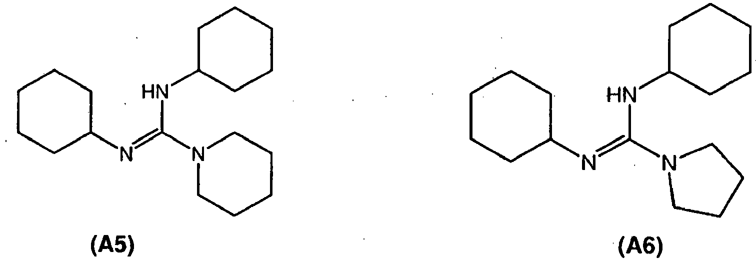

Les catalyseurs conformes à la présente invention sont non toxiques, contrairement aux catalyseurs à base d'alkylétain. De plus, ils permettent d'atteindre, aussi bien dans des compositions monocomposantes que bicomposantes, des vitesses de polycondensation des silicones aussi élevées voire meilleures que celles obtenues avec ces catalyseurs à base d'alkylétain.

### Description de la base silicone B :

Les bases silicones utilisées dans la présente invention réticulant et durcissant par des réactions de polycondensation sont bien connues. L'expression « base silicone » ne comprend pas les compositions contenant des polymères organiques ayant des groupements silicés réticulables par polycondensation.

Les bases silicones selon l'invention sont décrites en détail en particulier dans de nombreux brevets et elles sont disponibles dans le commerce.

Ces bases silicones peuvent être mono-composantes, c'est-à-dire conditionnées en un seul emballage, et stables au stockage en l'absence d'humidité, durcissables en présence d'humidité, en particulier d'humidité apportée par l'air ambiant ou par de l'eau générée au sein de la base lors de son emploi.

En dehors des bases mono-composantes peuvent être utilisées des bases bi-composantes, c'est-à-dire conditionnées en deux emballages, qui durcissent dès l'incorporation du catalyseur de polycondensation selon l'invention. Elles sont conditionnées après incorporation du catalyseur en deux fractions séparées, l'une des fractions pouvant ne renfermer par exemple que le catalyseur selon l'invention ou un mélange avec l'agent réticulant.

La base silicone **B** utilisée pour réaliser la composition selon l'invention comprend:
- au moins une huile polyorganosiloxane **C** susceptible de réticuler par polycondensation en un élastomère;
- éventuellement au moins un agent de réticulation **D;**
- éventuellement au moins un promoteur d'adhérence **E;** et
- éventuellement au moins une charge minérale siliceuse, organique et/ou non siliceuse **F.**

L'huile polyorganosiloxane **C** est de préférence un polymère α,ω-dihydroxypolydiorganosiloxane, de viscosité comprise entre 50 et 5 000 000 mPa.s à 25°C et l'agent de réticulation **D** est de préférence un composé organosilicié portant plus de deux groupes hydrolysables liés aux atomes de silicium par molécule. L'huile polyorganosiloxane **C** peut également être fonctionnalisée au niveau de ses extrémités par des radicaux hydrolysables obtenu par condensation d'un précurseur porteur de fonctions hydroxyles avec un silane réticulant porteur de radicaux hydrolysables.

Comme agent de réticulation **(D)** on peut citer :
- les silanes de formule générale suivante :

   R¹ₖSi(OR²)₍₄₋ₖ₎

   dans laquelle les symboles R², identiques ou différents, représentent des radicaux alkyles ayant de 1 à 8 atomes de carbone, tels que les radicaux méthyle, éthyle, propyle, butyle, pentyle, éthyl-2 hexyle, des radicaux oxyalkylènes en C₃-C₆, le symbole R¹ représente un groupe hydrocarboné aliphatique saturé ou insaturé, linéaire ou ramifié, un groupe carbocyclique, saturé ou insaturé et/ou aromatique, monocyclique ou polycyclique et k est égal à 0, 1 ou 2 ; et
- les produits d'hydrolyse partielle de ce silane.

Comme exemple de radicaux alcoxyalkylène en C₃-C₆ on peut citer les radicaux suivants :

CH₃OCH₂CH₂-

CH₃OCH₂CH(CH₃)-

CH₃OCH(CH₃)CH₂-

C₂H₅OCH₂CH₂CH₂-

Lé symbole R¹ représente un radical hydrocarbonés en C₁-C₁₀ englobant:
- les radicaux alkyles en C₁-C₁₀ tels que les radicaux méthyle, éthyle, propyle, butyle, pentyle, éthyl-2 hexyle, octyle, décyle,
- les radicaux vinyle, allyle, et
- les radicaux cycloalkyles ce C₅-C₈ tels que les radicaux phényle, tolyle et xylyle.

Les agents de réticulation **D** sont des produits accessibles sur le marché des silicones ; de plus leur emploi dans les compositions durcissant dès la température ambiante est connu ; il figure notamment dans les brevets français FR-A-1 126 411, FR-A-1 179 969, FR-A-1 189 216, FR-A-1 198 749, FR-A-1 248 826, FR-A-1 314 649, FR-A-1 423 477, FR-A-1 432 799 et FR-A-2 067 636.

Parmi les agents de réticulation **D,** on préfère plus particulièrement les alkyltrialcoxysilanes, les silicates d'alkyle et les polysilicates d'alkyle, dans lesquels les radicaux organiques sont des radicaux alkylés ayant de 1 à 4 atomes de carbone.

Comme autres exemples d'agents de réticulation **D** qui peuvent être utilisés, on cite plus particulièrement les silanes suivants :
- le propyltriméthoxysilane,
- le méthyltriméthoxysilane,
- l'éthyltriméthoxysilane,
- le vinyltriéthoxysilane,
- le méthyltriéthoxysilane,
- le propyltriéthoxysilane,
- le tétraéthoxysilane,
- le tétrapropoxysilane,
- le 1,2-bis(triméthoxysilyl)éthane,
- le 1,2-bis(triéthoxysilyl)éthane, et
- le tétraisopropoxysilane,
ou encore: CH₃Si(OCH₃)₃ ; C₂H₅Si(OC₂H₅)₃ ; C₂H₅Si(OCH₃)₃ CH₂=CHSi(OCH₃)₃; CH₂=CHSi(OCH₂CH₂OCH₃)₃ C₆H₅Si(OCH₃)₃ ; [CH₃][OCH(CH₃)CH₂OCH₃]Si[OCH₃]₂ Si(OCH₃)₄ ; Si(OC₂H₅)₄; Si(OCH₂CH₂CH₃)₄; Si(OCH₂CH₂CH₂CH₃)₄ Si(OC₂H₄OCH₃)₄ ; CH₃Si(OC₂H₄OCH₃)₃ ; ClCH₂Si(OC₂H₅)₃ ;

Comme autres exemples d'agent de réticulation **D,** on peut citer le polysilicate d'éthyle, ou le polysilicate de n-propyle.

On utilise généralement de 0,1 à 60 parties en poids d'agent de réticulation **D** pour 100 parties en poids de polyorganosiloxane **C** susceptible de réticuler par polycondensation en un élastomère.

Ainsi la composition selon l'invention peut comprendre au moins un promoteur d'adhérence E tel que par exemple les composés organosiliciques portant à la fois :
(1) un ou des groupes hydrolysables liés à l'atome de silicium, et
(2) un ou des groupes organiques substitués par des radicaux comprenant un atome d'azote ou choisis dans le groupe des radicaux (méth)acrylate, époxy, et alcényle, et plus préférentiellement encore dans le groupe constitué par les composés suivants pris seul ou en mélange:
   vinyltriméthoxysilane (VTMO),
   3-glycidoxypropyl-triméthoxysilane (GLYMO),
   méthacryloxypropyltriméthoxysilane (MEMO),
   [H₂N(CH₂)₃]Si(OCH₂CH₂CH₃)₃,
   [H₂N(CH₂)₃]Si(OCH₃)₃
   [H₂N(CH₂)₃]Si(OC₂H₅)₃
   [H₂N(CH₂)₄]Si(OCH₃)₃
   [H₂NCH₂CH(CH₃)CH₂CH₂]SiCH₃(OCH₃)₂
   [H₂NCH₂]Si(OCH₃)₃
   [n-C₄H₉-HN-CH₂]Si(OCH₃)₃
   [H₂N(CH₂)₂NH(CH₂)₃]Si(OCH₃)₃
   [H₂N(CH₂)₂NH(CH₂)₃]Si(OCH2CH₂OCH₃)₃
   [CH₃NH(CH₂)₂NH(CH₂)₃]Si(OCH₃)₃
   [H(NHCH₂CH₂)₂NH(CH₂)₃]Si(OCH₃)₃

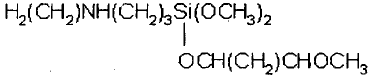

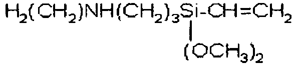

   ou des oligomères polyorganosiloxaniques contenant de tels groupes organiques à une teneur supérieure à 20%.

Pour les bases mono- et bi-composantes, on utilise comme charges minérales **F** des produits très finement divisés dont le diamètre particulaire moyen est inférieur à 0,1 µm. Parmi ces charges figurent les silices de combustion et les silices de précipitation; leur surface spécifique BET est généralement supérieure à 40 m²/g. Ces charges peuvent également se présenter sous la forme de produits plus grossièrement divisés, de diamètre particulaire moyen supérieur à 0,1 µm. Comme exemples de telles charges, on peut citer le quartz broyé, les silices de diatomées, le carbonate de calcium, l'argile calcinée, l'oxyde de titane du type rutile, les oxydes de fer, de zinc, de chrome, de zirconium, de magnésium, les différentes formes d'alumine (hydratée ou non), le nitrure de bore, le lithopone, le métaborate de baryum, le sulfate de baryum, les microbilles de verre; leur surface spécifique est généralement inférieure à 30 m²/g.

Ces charges peuvent avoir été modifiées en surface par traitement avec les divers composés organosiliciques habituellement employés pour cet usage. Ainsi ces composés organosiliciques peuvent être des organochlorosilanes, des diorganocyclopolysiloxanes, des hexaorganodisiloxanes, des hexaorganodisilazanes ou des diorganocyclopolysilazanes (brevets français FR-A-1 126 884, FR-A-1 136 885, FR-A-1 236 505, brevet anglais GB-A-1 024 234). Les charges traitées renferment, dans la plupart des cas, de 3 à 30 % de leur poids de composés organosiliciques. Les charges peuvent être constituées d'un mélange de plusieurs types de charges de granulométrie différente; ainsi par exemple, elles peuvent être constituées de 30 à 70 % de silices finement divisées de surface spécifique BET supérieure à 40 m² /g et de 70 à 30 % de silices plus grossièrement divisées de surface spécifique inférieure à 30 m²/g.

L'introduction des charges a pour but de conférer de bonnes caractéristiques mécaniques et rhéologiques aux élastomères découlant du durcissement des compositions conformes à l'invention.

En combinaison avec ces charges peuvent être utilisés des pigments minéraux et/ou organiques ainsi que des agents améliorant la résistance thermique (sels et oxydes de terres rares tels que les oxydes et hydroxydes cériques) et/ou la résistance à la flamme des élastomères. Par exemple on peut utiliser les coktails d'oxyde décrits dans la demande internationale WO 98/29488. Parmi les agents améliorant la résistance à la flamme peuvent être cités les dérivés organiques halogénés, les dérivés organiques du phosphore, les dérivés du platine tels que l'acide chloroplatinique (ses produits de réaction avec des alcanols, des éthers-oxydes), les complexes chlorure platineux-oléfines. Ces pigments et agents représentent ensemble au plus 20 % du poids des charges.

D'autres agents auxiliaires et additifs usuels peuvent être incorporés à la composition selon l'invention; ceux-ci sont choisis en fonction des applications dans lesquelles sont utilisées lesdites compositions.

La base silicone utilisée pour réaliser la composition selon l'invention peut comprendre:
- 100 parties d'huile polyorganosiloxane **C** susceptible de réticuler par polycondensation en un élastomère ;
- 0 à 20 parties d'un agent de réticulation **D;**
- 0 à 20 parties d'un promoteur d'adhérence **E;** et
- 0 à 50 parties de charge **F.**

Outre les constituants principaux, des polymères polyorganosiloxanes linéaires non réactifs G peuvent être introduits dans le dessein d'agir sur les caractéristiques physiques des compositions conformes à l'invention et/ou sur les propriétés mécaniques des élastomères issus du durcissement de ces compositions.

Ces polymères polyorganosiloxanes linéaires non réactif **G** sont bien connus; ils comprennent plus spécialement: des polymères α,ω-bis(triorganosiloxy)diorganopolysiloxanes de viscosités d'au moins 10 mPa.s à 25 °C, formés essentiellement de motifs diorganosiloxy et d'au plus 1 % de motifs monoorganosiloxy et/ou siloxy, les radicaux organiques liés aux atomes de silicium étant choisis parmi les radicaux méthyle, vinyle et phényle, 60 % au moins de ces radicaux organiques étant des radicaux méthyle et 10 % au plus étant des radicaux vinyle. La viscosité de ces polymères peut atteindre plusieurs dizaines de millions de mPa.s à 25°C; ils comprennent donc des huiles d'aspect fluide à visqueux et des gommes molles à dures. Ils sont préparés selon les techniques usuelles décrites plus précisément dans les brevets français FR-A-978 058, FR-A-1 025 150, FR-A-1 108 764, FR-A-1 370 884. On utilise de préférence les huiles α,ω-bis(triméthylsiloxy) diméthylpolysiloxanes de viscosité allant de 10 mPa.s à 1 000 mPa.s à 25°C. Ces polymères qui jouent le rôle de plastifiants peuvent être introduits à raison d'au plus 70 parties, de préférence de 5 à 20 parties, pour 100 parties de d'huile polyorganosiloxane **C** susceptible de réticuler par polycondensation.

Les compositions selon l'invention peuvent en outre avantageusement comprendre au moins une résine silicone **H.** Ces résines silicones sont des polymères organopolysiloxanes ramifiés bien connus et disponibles dans le commerce. Elles présentent, par molécule, au moins deux motifs différents choisis parmi ceux de formule R"'₃SiO_{1/2} (motif M), R"'₂SiO_{2/2} (motif D), R"'SiO_{3/2} (motif T) et SiO_{4/2} (motif Q). Les radicaux R"' sont identiques ou différents et sont choisis parmi les radicaux alkyles linéaires ou ramifiés, les radicaux vinyle, phényle, trifluoro-3,3,3 propyle. De préférence, les radicaux alkyles présentent de 1 à 6 atomes de carbone inclus. Plus particulièrement, on peut citer comme radicaux R alkyle, les radicaux méthyle, éthyle, isopropyle, tertiobutyle et n-hexyle. Ces résines sont de préférence hydroxylées et ont dans ce cas une teneur pondérale en groupe hydroxyle comprise entre 5 et 500 meq/100 g.

Comme exemple de résines, on peut citer les résines MQ, les résines MDQ, les résines TD et les résines MDT.

Pour fabriquer les compositions conformes à l'invention, il est nécessaire dans le cas des compositions mono-composantes d'utiliser un appareillage qui permette de mélanger intimement à l'abri de l'humidité, avec et sans apport de chaleur, les divers constituants fondamentaux auxquels sont éventuellement ajoutés les adjuvants et additifs précités. Tous ces ingrédients peuvent être chargés dans l'appareillage selon un ordre quelconque d'introduction. Ainsi il est possible de mélanger tout d'abord les huiles organopolysiloxanes **C** et les charges **F** et d'ajouter ensuite à l'empâtage obtenu les réticulants **D,** les composés **E** et le catalyseur selon l'invention. Il est également possible de mélanger les huiles **C,** les réticulants **D,** les composés **E** et les charges **F** et d'ajouter ultérieurement le catalyseur selon l'invention. Au cours de ces opérations, les mélanges peuvent être chauffés à une température comprise dans l'intervalle 50-180°C sous la pression atmosphérique ou sous une pression réduite afin de favoriser le départ de matières volatiles.

Les compositions mono-composantes, conformes à l'invention, utilisées telles quelles, c'est-à-dire non diluées, ou sous forme de dispersions dans des diluants, sont stables au stockage en l'absence d'eau et durcissent dès les basses températures (après départ des solvants dans le cas des dispersions) en présence d'eau pour former des élastomères.

Après le dépôt des compositions telles quelles, sur des substrats solides, en atmosphère humide, on constate qu'un processus de durcissement en élastomères se met en oeuvre, il s'effectue de l'extérieur à l'intérieur de la masse déposée. Une peau se forme d'abord en surface puis la réticulation se poursuit en profondeur. La formation complète de la peau, qui se traduit par un toucher non collant de la surface, demande une période de temps de quelques minutes; cette période dépendant du taux d'humidité relative de l'atmosphère entourant les compositions et de la faculté de réticulation de celles-ci.

Par ailleurs le durcissement en profondeur des couches déposées, qui doit être suffisant pour permettre le démoulage et la manipulation des élastomères formés, nécessite une période de temps plus longue. En effet, cette période dépend non seulement des facteurs cités ci-dessus pour la formation du toucher non collant mais aussi de l'épaisseur des couches déposées, laquelle épaisseur s'échelonne généralement entre 0,5 mm et plusieurs centimètres. Les compositions mono-composantes peuvent être employées pour de multiples applications comme le jointoiement dans l'industrie du bâtiment, l'assemblage des matériaux les plus divers (métaux, matières plastiques, caoutchoucs naturels et synthétiques, bois, carton, faïence, brique, céramique, verre, pierre, béton, éléments de maçonnerie), l'isolation de conducteurs électriques, l'enrobage de circuits électroniques, la préparation de moules servant à la fabrication d'objets en résines ou mousses synthétiques.

La fabrication des compositions bi-composantes conformes à l'invention s'effectue également par mélange des divers constituants dans des appareils appropriés. Pour obtenir des compositions homogènes, il est préférable de mélanger tout d'abord les polymères **A** avec les charges **C** ; l'ensemble peut être chauffé au moins 30 minutes à une température supérieure à 80°C, de manière à parfaire le mouillage des charges par les huiles. Au mélange obtenu, porté de préférence à une température inférieure à 80°C, par exemple de l'ordre de la température ambiante, peuvent être ajoutés les autres constituants, c'est-à-dire les agents réticulants, le catalyseur et éventuellement des additifs et adjuvants divers et même de l'eau.

Les compositions conformes à l'invention peuvent être employées pour de multiples applications comme le jointoiement et/ou le collage dans l'industrie du bâtiment, l'industrie du transport (exemples : automobile, aérospatiale, ferroviaire, maritime et aéronautique), l'assemblage des matériaux les plus divers (métaux, matières plastiques, caoutchoucs naturels et synthétiques, bois, cartons, polycarbonate, faïence, brique, céramique, verre, pierre, béton et éléments de maçonnerie), l'isolation de conducteurs électriques, l'enrobage de circuits électroniques et la préparation de moules servant à la fabrication d'objets en résines ou mousses synthétiques.

Ainsi, un autre objet de l'invention consiste en un système bicomposant précurseur de la composition organopolysiloxanique selon l'invention et telle que définie ci-dessus et vulcanisable en élastomère silicone par des réactions de polycondensation et caractérisé en ce qu'il se présente en deux parties **P1** et **P2** distinctes destinées à être mélangées pour former ladite composition, et en ce que l'une de ces parties comprend le catalyseur selon l'invention et tel que défini ci-dessus comme catalyseur de la réaction de polycondensation d'organopolysiloxanes et l'agent de réticulation **D** alors que l'autre partie est exempte des espèces précitées et comprend:
- pour 100 parties en poids de l'huile (ou des huiles) polyorganosiloxane(s) **C** susceptible(s) de réticuler par polycondensation en un élastomère, et
- de 0.001 à 10 partie(s) en poids d'eau.

Un autre objet de l'invention consiste aussi en une composition polyorganosiloxanique monocomposante stable au stockage en l'absence d'humidité et réticulant, en présence d'eau, en élastomère, caractérisée en ce qu'elle comprend:
- au moins un polyorganopolysiloxane linéaire réticulable présentant des extrémités fonctionnalisées de type alcoxy, oxime, acyle et /ou énoxy, de préférence alcoxy,
- une charge, et
- le catalyseur de la réaction de polycondensation selon l'invention et tel que défini ci-dessus.

Des bases mono-composantes sont décrites en détail par exemple dans les brevets EP 141 685, EP 147 323, EP 102 268, EP 21 859, FR 2 121 289 et FR 2 121 631, cités en référence.

A ces bases mono-composantes peuvent être ajoutés des promoteurs d'adhérence **E** choisis par exemple parmi les composés organosiliciés portant à la fois, d'une part, des groupes organiques substitués par des radicaux choisis dans le groupe des radicaux amino, uréido, isocyanate, époxy, alkènyle, isocyanurate, hydentoile, guanidino et mercaptoester et, d'autre part des groupes hydrolysables, en général des groupes alcoxy liés aux atomes de silicium. Des exemples de tels agents d'adhérence sont décrits dans les brevets américains US 3 517 001, US 4 115 356, US 4 180 642, US 4 273 698, US 4 356 116 et dans les brevets européens EP 31 996 et EP 74 001.

Des bases bi-composantes sont décrites en détail par exemple dans les brevets EP 118 325, EP 117 772, EP 10 478, EP 50 358, EP 184 966, US 3 801 572 et US 3 888 815, cités comme référence.

D'autres avantages et caractéristiques de la présente invention apparaîtront à la lecture des exemples suivants donnés à titre illustratif nullement limitatif.

### EXEMPLES:

### I) Préparation des catalyseurs selon l'invention

### a) 1-butyl-2,3-diisopropylguanidine (A1) :

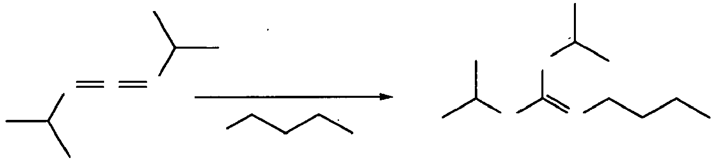

Un mélange de 33 g de N-butylamine (0,45 mol) et de 19 g de diisopropylcarbodiimide (0,15 mol) est chauffé à reflux 3h30. L'analyse par CPG montre alors une conversion supérieure à 99.5 % de la diisopropylcarbodiimide. Le mélange final incolore est concentré à 60°C sous 20 mbar pendant 2 h pour donner 29 g d'un liquide incolore et pratiquement inodore de faible viscosité, correspondant à la guanidine attendue (rendement 96.7 %).
RMN ¹H/CDCl₃ (ppm) : 0.93 (3 H, t), 1.14 (12 H, d), 1.37 (2 H, sex), 1.52 (2 H, quint), 3.01 (2 H, t), 3.57 (2 H, m).

### b) 1-butyl-2,3-diisopropyl-1-méthylguanidine (A3) :

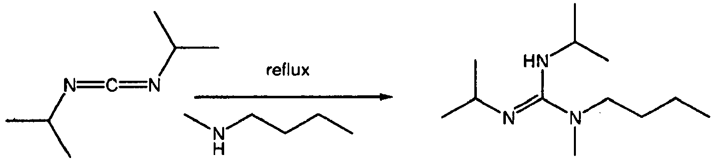

Un mélange de 32.68 g de N-butyl-N-méthylamine (0,375 mol) et de 23.66 g de diisopropylcarbodiimide (0,1875 mol) est chauffé à reflux 3h. L'analyse par CPG montre alors une conversion supérieure à 99.5 % de la diisopropylcarbodiimide. Le mélange final incolore est concentré à 60°C sous 5 mbar pendant 2 h pour donner 40 g d'un liquide incolore et pratiquement inodore de faible viscosité, correspondant à la guanidine attendue (rendement 100 %).
RMN ¹H/CDCl₃ (ppm) : 0.88 (3 H, t), 1.06 (12 H, d), 1.26 (2 H, sex), 1.46 (2 H, quint), 2.67 (3 H, s), 3.05 (2 H, t), 3.35 (2 H, m).

### c) 1-butyl-2,3-dicyclohexylguanidine (A2) RN-CAS = 60006-40-8

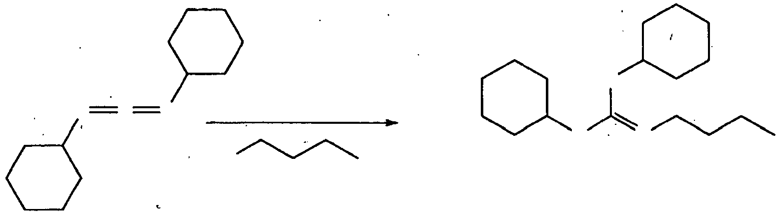

Un mélange de 15.69 g de N-butylamine (0,214 mol) et de 22.13 g de dicyclohexylcarbodiimide (0,107 mol) est chauffé à reflux 2h. L'analyse par CPG montre alors une conversion supérieure à 99.6 % de la dicyclohexylcarbodiimide. Le mélange final incolore est concentré à 60°C sous 1 mbar pendant 2 h pour donner 29.7 g d'un liquide incolore et pratiquement inodore moyennement visqueux, correspondant à la guanidine attendue (rendement 99 %).

### d) 1-butyl-2,3-dicyclohexyl-1-méthylguanidine (A4):

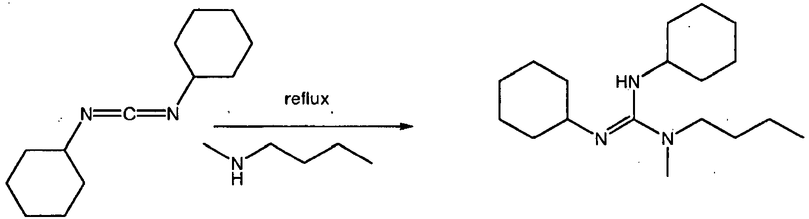

Un mélange de 17.78 g de N-butyl-N-méthylamine (0,204 mol) et de 21.05 g de dicyclohexylcarbodiimide (0,102 mol) est chauffé à reflux 3h. L'analyse par CPG montre alors une conversion supérieure à 99.5 % de la dicyclohexylcarbodiimide. Le mélange final incolore est concentré à 60°C sous 1 mbar pendant 2 h pour donner 29.9 g d'un liquide incolore et pratiquement inodore moyennement visqueux, correspondant à la guanidine attendue (rendement 99.7 %).
RMN ¹H/CDCl₃ (ppm) : 0.89 (3 H, t), 1-1.4 (10 H, m), 1.47 (2 H, quint), 1.5-2 (12 H, plusieurs m), 2.67 (3 H, s), 2.90 (1 H, m), 2.97 (1 H, m), 3.06 (2 H, t).

### e) 1,2-Dicyclohexyl-3-pipéridylguanidine (A5) RN-CAS 60006-25-9 :

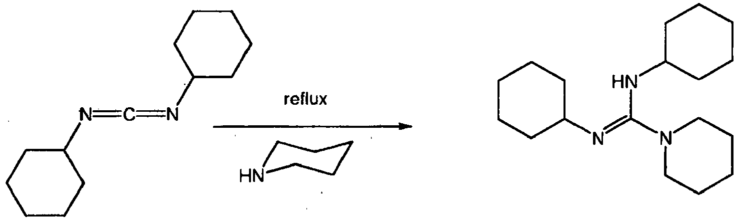

Un mélange de 11.69 g de pipéridine (0,137 mol) et de 14.16 g de dicyclohexylcarbodiimide (0,0686 mol) est chauffé à reflux 3h30. L'analyse par CPG montre alors une conversion supérieure à 99.7 % de la dicyclohexylcarbodiimide. Le mélange final incolore est concentré à 60°C sous 1 mbar pendant 2 h pour donner 19.9 g d'un liquide incolore et pratiquement inodore trés visqueux, correspondant à la guanidine attendue (rendement 99.5 %).

### f) 1,2-Dicyclohexyl-3-pyrrolidylguanidine (A6) RN-CAS 60006-28-2 :

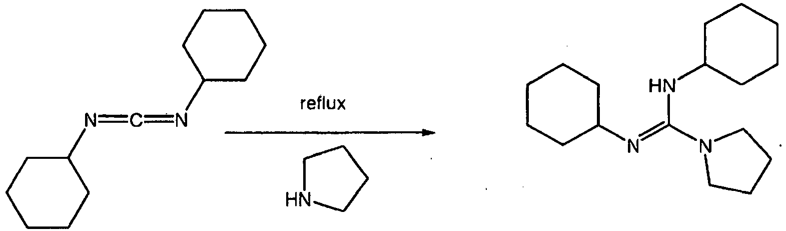

Un mélange de 19.2 g de pyrrolidine (0,27 mol) et de 18.6 g de dicyclohexylcarbodiimide (0,09 mol) est chauffé à reflux 4h. L'analyse par CPG montre alors une conversion supérieure à 99.8 % de la dicyclohexylcarbodiimide. Le mélange final incolore est concentré à 60°C sous 1 mbar pendant 1 h pour donner 24.9 g d'un liquide incolore et pratiquement inodore moyennement visqueux, correspondant à la guanidine attendue (rendement 99.6 %).

### II. Préparation de compositions mono-composantes

### a) Test en empâtage - réticulant vinyltriméthoxysilane

L'empâtage utilisé est préparé comme suit : à un mélange de 3464 g d'une huile α,ω-dihydroxylée de viscosité de 20000 centipoises contenant 0.066% d'OH, et de 120 g de vinyltriméthoxysilane sous agitation, est ajouté 16 g d'une solution de lithine à 2 % en poids dans le méthanol, puis après 5 min, 400 g de silice de pyrogénation AE55 sont ajoutés. Le mélange est dévolatilisé sous vide puis stocké à l'abri de l'humidité.

Pour chaque test, le catalyseur testé est mélangé à 50 g de cet empâtage, puis le potentiel catalytique est évalué de 3 façons :
- le temps de formation de peau (TFP), temps au bout duquel on observe une réticulation en surface, sur film de 2 mm,
- la persistance d'un toucher collant à 48h,
- la dureté (en Shore A) d'un cordon de 6 mm d'épaisseur dans des conditions régulées (23°C et 50% d'humidité relative) et sur des temps croissants (2, 3, 4, 7 et 14 jours), ainsi que après 7 jours (7J) à température ambiante (TA) suivi de 7 jours à 100°C. Dans les tableaux de résultats, le symbole « > » correspond aux valeurs de dureté mesurées sur la partie supérieure du cordon et le symbole « < » correspond aux valeurs de dureté mesurées sur la partie inférieure du cordon moins exposée à l'air ambiant que la partie supérieure.

Différents catalyseurs selon l'invention ont été testés. A titre comparatif, comme précédemment, ont été également testés:
- un catalyseur à base d'étain: le dilaurate de dibutylétain (DLBDE), et
- la 1,1,3,3-tétraméthylguanidine (TMG) qui est décrite dans la demande internationale WO 2004/020525. Les résultats sont indiqués dans le tableau I ci-dessous:

**Tableau I.**

| **Référence** | **Structure** | **mol/Sn** | **% poids** | **TFP baton** | **Toucher collant** | **Dureté Shore A sur 6mm** | | | | | | | | | | | |
|---|---|---|---|---|---|---|---|---|---|---|---|---|---|---|---|---|---|
| | | | | | **à** | **2J** | | **3J** | | **4J** | | **7J** | | **14J** | | **7JTA + 7J 100°C** | |
| | | | | **min** | **48h** | **>** | **<** | **>** | **<** | **>** | **<** | **>** | **<** | **>** | **<** | **>** | **<** |
| **Comparatif (DLDBE)** | | 1 | 0,88 | 10 | non | 34 | 26 | 35 | 30 | 35 | 30 | 35 | 29 | 35 | 30 | 35 | 28 |
| **Comparatif (TMG)** | 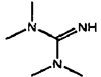 | 4 | 0,64 | 2 | oui | 2 | 1 | 2 | 1 | / | | 4 | 3 | 2 | 1 | 1 | 1 |
| **Invention (A1)** | 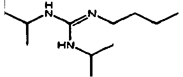 | 3 | 0,84 | 1 | non | 36 | 26 | 36 | 28 | 37 | 28 | 38 | 28 | 38 | 30 | 34 | 27 |
| | | 1,5 | 0,42 | 2 | non | 35 | 22 | 36 | 26 | 36 | 27 | 37 | 28 | 39 | 28 | 36 | 26 |
| | | 0,75 | 0,21 | 2 | oui | 30 | 4 | 32 | 6 | 33 | 9 | 35 | 14 | 37 | 21 | 35 | 21 |
| **Invention (A2)** | 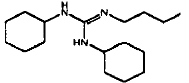 | 1,5 | 0,59 | 1 | non | 34 | 27 | 35 | 28 | 35 | 30 | 36 | 30 | 37 | 31 | 31 | 25 |
| **Invention (A3)** | 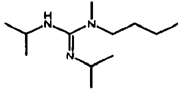 | 3 | 0,9 | 3 | non | 35 | 27 | 36 | 29 | 36 | 29 | 35 | 29 | 37 | 31 | 37 | 30 |
| | | 1,5 | 0,46 | 5 | non | 35 | 21 | 37 | 28 | 37 | 30 | 37 | 30 | 39 | 31 | 37 | 31 |
| | | 0,75 | 0,23 | 6 | oui | 31 | 12 | 33 | 21 | 35 | 24 | 36 | 27 | 39 | 30 | 37 | 27 |
| | | 0,375 | 0,11 | 12 | oui | 22 | 4 | 26 | 6 | 28 | 10 | 28 | 13 | 32 | 23 | 31 | 21 |
| **Invention (A4)** | 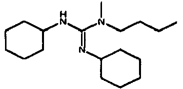 | 0,75 | 0,31 | 5 | non | 35 | 24 | 36 | 28 | 37 | 29 | 37 | 29 | 37 | 30 | 37 | 30 |
| | | 0,375 | 0,15 | 7 | non | 30 | 13 | 32 | 19 | 33 | 23 | 34 | 26 | 35 | 28 | 35 | 29 |

La 1,1,3,3-tétraméthylguanidine (TMG) ne permet pas la réticulation de l'huile silicone, pourtant à des concentrations molaires bien supérieures aux guanidines selon l'invention **(A1)** à **(A4).** Les guanidines 1,2,3-trisubstituées **(A1)** et **(A2)** conduisent à des temps de formation de peau très courts et à des élastomères. Les guanidines 1,2,3,3-tétrasubstituées **(A3)** et **(A4)** permettent non seulement en adaptant les teneurs jusqu'à des valeurs très faibles de moduler les durées des temps de formation de peau tout en obtenant des élastomères très stables thermiquement et de niveaux de réticulation légèrement plus élevés qu'avec le catalyseur à l'étain (DLDBE). Ces résultats montrent que les catalyseurs selon l'invention **(A1)** à **(A4),** non toxiques, conduisent à une catalyse plus efficace que les catalyseurs à base d'étain (DLDBE) et que la tétraméthylguanidine (TMG). Les catalyseurs selon l'invention peuvent donc avantageusement remplacer les catalyseurs existants.

### b) Test en empâtage - réticulant vinyltriéthoxysilane

L'empâtage utilisé est préparé comme suit : à un mélange de 857.5 g d'une huile α,ω-dihydroxylée de viscosité de 20000 centipoises contenant 0.066% d'OH, et de 38.5 g de vinyltriéthoxysilane sous agitation est ajouté 4 g d'une solution de lithine à 4 % en poids dans le méthanol, puis après 20 min, 100 g de silice de pyrogénation AE55 sont ajoutés. Le mélange est dévolatilisé sous vide puis stocké à l'abri de l'humidité. Pour chaque test, le catalyseur testé est mélangé à 50 g de cet empâtage, puis le potentiel catalytique est évalué de la même façon que précédemment.

Différents catalyseurs selon l'invention ont été testés.
A titre comparatif, comme précédemment, a été également testé:
- un catalyseur à base d'étain: le dilaurate de dibutylétain (DLDBE).
Les résultats sont indiqués dans le tableau I ci-dessous:

**Tableau II**

| **Référence** | **Produit** | **nb éq** | **% poids** | **TFP baton** | **Toucher collant** | **Dureté Shore A sur 6mm** | | | | | | | | | | | |
|---|---|---|---|---|---|---|---|---|---|---|---|---|---|---|---|---|---|
| | | **1 éq = 0,70 mM** | | | **à** | **2J** | | **3J** | | **4J** | | **7J** | | **14J** | | **7J TA + 7J 100°C** | |
| | | | | **min** | **48h** | **>** | **<** | **>** | **<** | **>** | **<** | **>** | **<** | **>** | **<** | **>** | **<** |
| **Comparatif** | DLDBE | 1 | 0,9 | 60 | non | 10 | 7 | 16 | 12 | 20 | 16 | 26 | 22 | 32 | 29 | 33 | 29 |
| **Invention A1** | 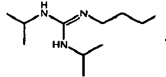 | 3 | 0,8 | 4 | non | 28 | 16 | 31 | 22 | 32 | 24 | 33 | 26 | 33 | 27 | 32 | 25 |
| | | 1,5 | 0,5 | 5 | non | 18 | 7 | 21 | 12 | 23 | 14 | 26 | 17 | 30 | 22 | 28 | 20 |
| **Invention A3** | 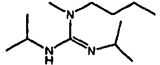 | 3 | 0,9 | 12 | non | 29 | 17 | 31 | 24 | 31 | 26 | 32 | 28 | 33 | 28 | 34 | 30 |
| | | 1,5 | 0,5 | 20 | oui | 18 | 9 | 23 | 16 | 26 | 21 | 31 | 25 | 33 | 30 | 32 | 27 |
| **Invention A4** | 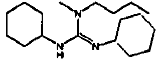 | 3 | 1.2 | 8 | non | 31 | 24 | 32 | 27 | 32 | 27 | 33 | 28 | 33 | 28 | 33 | 29 |
| | | 1,5 | 0,6 | 10 | non | 29 | 18 | 31 | 24 | 32 | 27 | 33 | 27 | 33 | 29 | 34 | 29 |

Le catalyseur à l'étain donne une bonne dureté mais seulement à 14 jours avec ce système beaucoup moins réactif. Le temps de formation de peau est par ailleurs prohibitif. Avec les catalyseurs selon l'invention, les temps de formation de peau sont courts et modulables selon la teneur et les cinétiques de dureté beaucoup plus rapides: des niveaux de dureté équivalents sont obtenus. Un élastomère ne dégageant pratiquement que de l'éthanol est donc permis avec les catalyseurs selon l'invention.

### III) Préparation de compositions bi-composantes

La comparaison de l'activité des catalyseurs selon l'invention par rapport au catalyseur habituel (bis-néodécanoate de diméthylétain - UL28, comparatif 1) et à la 1,1,3,3-tétraméthylguanidine (comparatif 2), est réalisée sur un système simplifié : à 25 g d'une huile ct,ù>dihydroxylée de viscosité de 14000 centipoises contenant 0.065% d'OH est mélangé 1.06 g de silicate d'éthyle "avancé" (= partiellement hydrolysé), puis la même quantité molaire (0.7 mmol) de catalyseur. On mesure le temps de travail ou temps de gel, puis les duretés d'un pion de 6 mm d'épaisseur dans des conditions régulées (23°C et 50% d'humidité relative) et sur des temps croissants. Dans le tableau suivant le symbole « > » correspond aux valeurs de dureté mesurées sur la partie supérieure du pion et le symbole « < » correspond aux valeurs de dureté mesurées sur la partie inférieure du pion moins exposée à l'air ambiant que la partie supérieure.
Les résultats sont indiqués dans le tableau III ci-dessous:

**Tableau III**

| catalyseur | Temps de travail | DSA 24h | | DSA 48h | | DSA 6 jours | | DSA (n jours) | | |
|---|---|---|---|---|---|---|---|---|---|---|
| | | > | < | > | < | > | < | > | < | (n j) |
| **Comparatif 1 (Sn)** | 6 min | 22.5 | 22.5 | Non mesurée | | Non mesurée | | 23 | 23 | (20 j) |
| **Comparatif 2 (TMG)** | 7 min | Pas de réticulation | | Pas de réticulation | | Pas de réticulation | | Pas de réticulation | | |
| **invention (A1)** | 30 s | 23 | 9 | Non mesurée | | 24 | 25 | 22 | 18 | (28 j) |
| **Invention (A3)** | 2min 30 s | 23 | 9 | 24 | 22 | Non mesurée | | 22 | 21 | (13 j) |
| **Invention (A4)** | 8-9 min | 21 | 19 | 21 | 21 | 24 | 24 | 23 | 23 | (13 j) |

La guanidine tétrasubstituée **(A4)** est particulièrement efficace avec un temps de travail et une cinétique de dureté tout à fait comparable avec la référence catalyseur étain.

### IV) RTV-II: composition silicone réticulant à température ambiante par réaction de polycondensation

On prépare les compositions bi-composantes détaillées dans le tableau IV. Les ingrédients listés ci-après sont les constituants utilisés pour les Exemples 1 à 4 et les Comparatifs 1 à 2 :
- a1 : huile polydiméthylsiloxane hydroxylée bloquée à chacune des extrémités de chaînes par un motif (CH₃)₂(OH)SiO_{0,5} ayant une viscosité de 14000 mPa.s à 25°C,
- a2 : huile polydiméthylsiloxane hydroxylée bloquée à chacune des extrémités de chaînes par un motif (CH₃)₂(OH)SiO_{0.5} ayant une viscosité de 750 mPa.s à 25°C,
- a3 : huile polydiméthylsiloxane hydroxylée bloquée à chacune des extrémités de chaînes par un motif (CH₃)₂(OH)SiO_{0.5} ayant une viscosité de 50 mPa.s à 25°C.
- b1 : silice de pyrogénation ayant une surface spécifique BET de 200 m²/g, traitée par de l'HMDZ, dispersée dans un mélange de a1 et d'une huile polydiméthylsiloxane bloquée à chacune des extrémités de chaînes par un motif (CH₃)₃SiO_{0.5},
- b2 : quartz broyé présentant un diamètre moyen de particules de 10 µm,
- c : eau,
- d1 : catalyseur selon l'invention (A4),
- d2 : catalyseur selon l'invention (A2),
- d3 : catalyseur selon l'invention (A3),
- d4 : comparatif 1,1,3,3-tétramethyl guanidine (TMG)
- d5 : comparatif Fomrez® catalyst UL-28 : dimethylbis[(1 oxoneodecyl)oxy]stannane = [C₉H₁₉COO]₂Sn(Me)₂
- e : polysilicate d'éthyle.

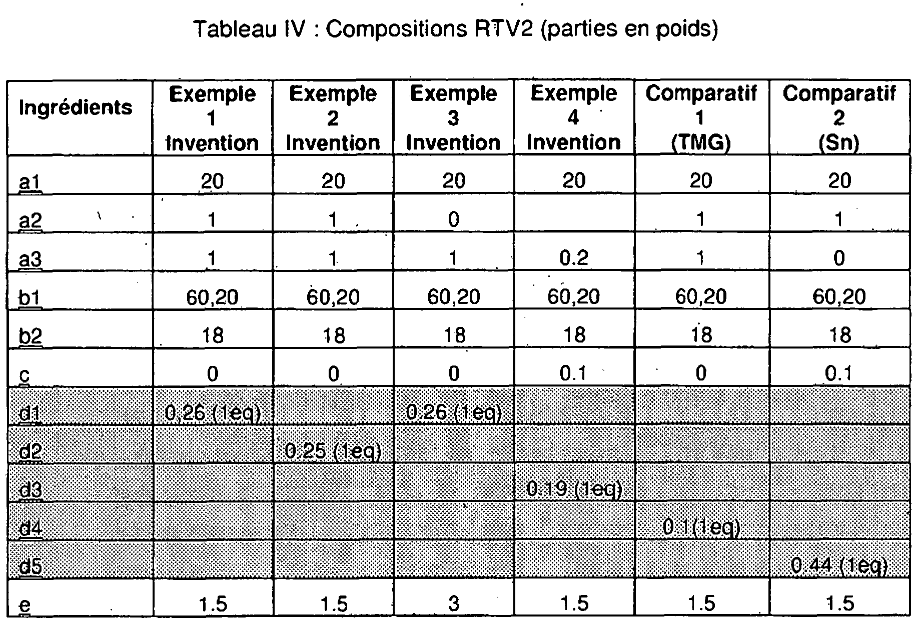

### 2) Evaluation des compositions

Les résultats du tableau V mettent en évidence l'efficacité des systèmes catalytiques revendiqués. De plus, l'exemple 1 démontre l'intérêt d'utiliser la guanidine 1 pour obtenir des élastomères ne contenant pas de produits toxiques et ayant un temps de travail adapté à l'application (c'est-à-dire un pot-life suffisamment long pour permettre son utilisation mais suffisamment court pour obtenir un objet manipulable au plus tard après 24h à 23°C).

**Tableau V - Cinétique de réticulation (Pot-life et Cinétique d'acquisition de Dureté Shore A)**

| | **Exemple 1 Invention** | **Exemple 2 Invention** | **Exemple 3 Invention** | **Exemple 4 Invention** | **Comparatif 1 (TMG)** |
|---|---|---|---|---|---|
| **Temps de travail (min.)** | 8min | 2min | 3min | 3min | 7 min |
| **DSA après 24h à 23°C (dessus/dessous)** | 21/19 | 20/20 | 17/18 | 12/13 | Non démoulable |
| **DSA après 2j à 23 °C (dessous)** | 22 | 22 | 26 | 20 | Non démoulable |
| **Aspect du pion réticulé** | Homogène | Homogène | Homogène | Homogène | Non démoulable |

- Dureté Shore A, notée DSA : mesures effectuées selon les indications de la norme ASTM-D2240 sur la partie supérieure et inférieure du pion.

Les résultats du tableau VI montrent que l'élastomère obtenu présente de bonnes propriétés mécaniques, similaires à celles obtenues avec un catalyseur à base d'étain.

**Tableau VI - Propriétés mécaniques**

| **Propriétés mécaniques** | **DSA 4j** | **Rd** | **RR** | **AR** |
|---|---|---|---|---|
| | **(dessous)** | **(N/mm)** | **(Mpa)** | **(%)** |
| **Exemple 1 - Invention** | 22 | 18 | 3.2 | 455 |
| **Comparatif 2 - (Sn)** | 22 | 20 | 3.5 | 400 |

- Rd = résistance à la déchirure (N/mm, mesures effectuées selon les indications de la norme ASTM-D624A).
- RR = résistance à la rupture (MPa, mesures effectuées selon les indications de la norme ASTM-D412 ou AFNOR-T-46002).
- AR = allongement à la rupture (%, mesures effectuées selon les indications de la norme ASTM-D412 ou AFNOR-T-46002).

**Tableau VII: DSA après traitement thermique à 220°C**

| **(Temps en h)** | **Exemple 1 Invention** | **Comparatif 2 (Sn)** |
|---|---|---|
| **0** | 26 | 23 |
| **4** | | |
| **7** | | 18 |
| **14** | | 23 |
| **21** | 25 | 25 |
| **22** | | |
| **28** | 24 | |
| **45** | | 28 |
| **54** | | |
| **69** | 23 | 28 |
| **86** | | |
| **120** | 23 | |

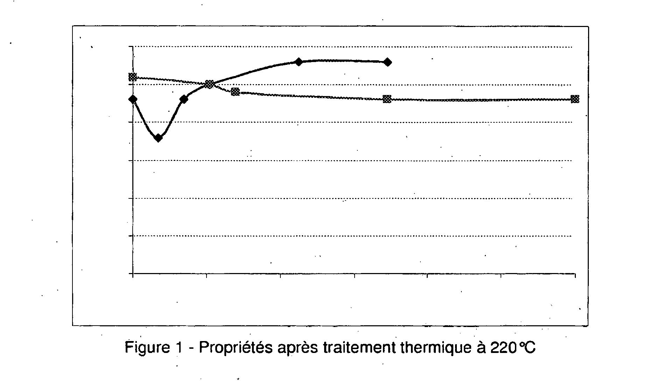

Le Tableau VII ci-dessus (voir aussi Figure 1) met également en évidence la très bonne tenue thermique des élastomères réticulés en présence du système catalytique revendiqué: en effet, la DSA de ces élastomères reste constante après un traitement thermique à 220°C, contrairement aux élastomères catalysés par des complexes de l'étain (à 220°C : perte de DSA, suivie d'une forte augmentation de DSA).

L'ensemble de ces résultats montre que le système catalytique selon l'invention conduit à l'obtention d'élastomères ayant des propriétés équivalentes ou supérieures au témoin, en particulier au niveau des propriétés de tenue thermique (pas d'évolution de DSA après un traitement thermique à 220°C de longue durée), accompagnée d'une cinétique très rapide compatible avec les cadences industrielles.

## Revendications

1. Composition organopolysiloxanique ne contenant pas de catalyseur métallique et **caractérisée en ce qu'**elle comprend d'une part une base silicone **B** comprenant au moins une huile polyorganosiloxane réticulable par réaction de polycondensation de manière à former un élastomère silicone et d'autre part, une quantité catalytiquement efficace d'au moins un catalyseur de polycondensation **A** qui est un composé organique non silylé et répondant à la formule générale **(I):** 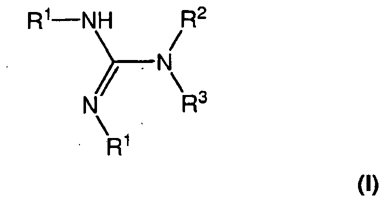 dans laquelle,
- les radicaux R¹, identiques ou différents, représentent, indépendamment l'un de l'autre, un groupe alkyle monovalent linéaire ou ramifié, un groupement cycloalkyle, un groupe (cycloalkyl)alkyle, le cycle étant substitué ou non et pouvant comprendre au moins un hétéroatome ou un groupement fluoroalkyle,
- le radical R² représente un atome d'hydrogène, un groupement alkyle monovalent linéaire ou ramifié, un groupement cycloalkyle, un groupement alkyle substitué par un cycle, substitué ou non et pouvant comprendre au moins un hétéroatome, un groupement aromatique un groupe - arylalkyle, un groupement fluoroalkyle, un groupement alkylamine ou alkylguanidine, et
- le radical R³ représente un groupement alkyle monovalent linéaire ou ramifié, un groupement cycloalkyle, un groupement alkyle substitué par un cycle, substitué ou non et pouvant comprendre au moins un hétéroatome, un groupement arylalkyle, fluoroalkyle, alkylamine ou alkylguanidine,
- lorsque le radical R² n'est pas un atome d'hydrogène, les radicaux R² et R³ peuvent être liés pour former un cycle aliphatique à 3, 4, 5, 6 ou 7 chaînons éventuellement substitué par un ou plusieurs substituants, et
- avec la condition supplémentaire que les radicaux R¹, R² et R³ ne comprennent pas d'atome de silicium.

2. Composition organopolysiloxanique selon la revendication 1, **caractérisée en ce que** le catalyseur de polycondensation **A** est un composé organique non silylé répondant à la formule générale **(I)** et dans laquelle:
- les radicaux R₁, identiques ou différents, et le radical R₃ sont choisis, indépendamment les uns des autres, dans le groupe constitué par: un radical isopropyle, un radical cyclohexyle et un radical alkyle monovalent linéaire ou ramifié en C₁ - C₁₂.
- le radical R₂ représente un atome d'hydrogène, un groupe alkyle monovalent linéaire ou ramifié, un groupe cycloalkyle, un groupe alkyle substitué par un cycle, substitué ou non et pouvant comprendre au moins un hétéroatome, un groupe arylalkyle, un groupe fluoroalkyle, un groupe alkylamine ou alkylguanidine, et
- lorsque le radical R² n'est pas un atome d'hydrogène, les radicaux R² et R³ peuvent être liés pour former un cycle aliphatique à 3, 4, 5, 6 ou 7 chaînons éventuellement substitué par un ou plusieurs substituants.

3. Composition organopolysiloxanique selon la revendication 2 dans laquelle le catalyseur de polycondensation **A** est un composé organique non silylé choisi parmi le groupe constitué par les composés **(A1)** à **(A6)** suivants:

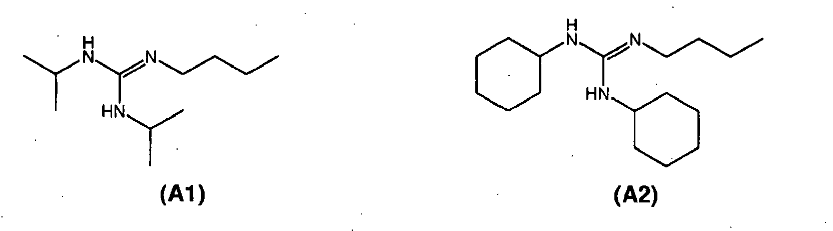

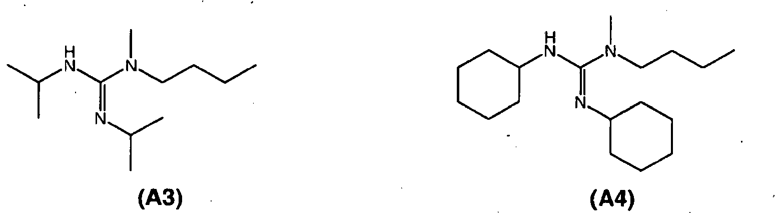

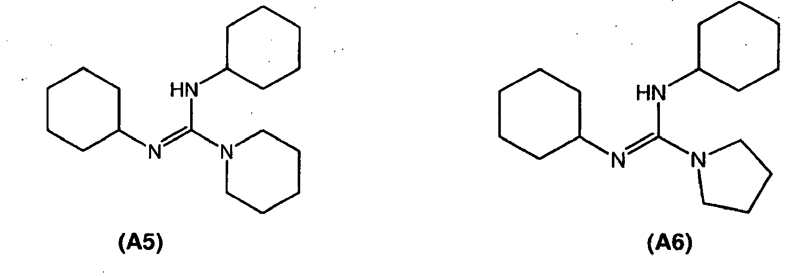

4. Composition selon l'une quelconque des revendications précédentes, **caractérisée en ce qu'**elle comprend outre une quantité catalytiquement efficace d'au moins un catalyseur de polycondensation **A** tel que défini selon l'une quelconque des revendications 1 à 3 et une base silicone **B** comprenant:
- au moins une huile polyorganosiloxane **C** susceptible de réticuler par polycondensation en un élastomère;
- éventuellement au moins un agent de réticulation **D;**
- éventuellement au moins un promoteur d'adhérence **E;** et
- éventuellement au moins une charge minérale siliceuse, organique et/ou non siliceuse **F.**

5. Composition organopolysiloxanique selon la revendication 4 vulcanisable en élastomère silicone par des réactions de polycondensation dans laquelle :
(a) la base silicone **B** comprend :
- pour 100 parties en poids d'au moins une huile polyorganosiloxane **C** susceptible de réticuler par polycondensation qui est un polymère réactif α,ω-dihydroxydiorganopolysiloxane dont les radicaux organiques sont des radicaux hydrocarbonés de préférence choisi parmi le groupe constitué par: les alkyles ayant de 1 à 20 atomes de carbone ; les cycloalkyles ayant de 3 à 8 atomes de carbone ; les alcényles ayant de 2 à 8 atomes de carbone et les cycloalcényles ayant de 5 à 8 atomes de carbone ;
- de 0,1 à 60 parties en poids d'au moins un agent de réticulation D choisi parmi le groupe constitué par: les polyalcoxysilanes, les produits provenant de l'hydrolyse partielle d'un polyalcoxysilane et les polyalcoxysiloxanes;
- de 0 à 60 parties en poids d'un promoteur d'adhérence **E** tel que décrit ci-dessus;
- de 0 à 250 parties en poids, de préférence de 5 à 200 parties en poids, d'au moins une charge minérale siliceuse, organique et/ou non siliceuse **F;**
- de 0,001 à 10 parties en poids d'eau,
- de 0 à 100 parties en poids d'au moins un polymère polyorganosiloxane linéaire non réactif **G** consistant dans un homopolymère ou copolymère linéaire dont, par molécule, les substituants organiques monovalents, identiques ou différents entre eux, liés aux atomes de silicium sont choisis parmi les radicaux alkyles, cycoalkyles, alcényles, aryles, alkylarylènes et arylalkylènes,
- de 0 à 20 parties en poids d'une base colorante ou d'un agent de coloration **H,**
- de 0 à 70 parties en poids de résines polyorganosiloxanes **I,** et
- de 0 à 20 parties d'additifs auxiliaires **J** connus de l'homme de métier, tels que des agents plastifiant ; des ralentisseurs de réticulation, des huiles minérales, des agents antimicrobien, des additifs de tenue thermique tels que les oxydes de titane, de fer ou de cérium, et
(b) de 0,1 à 50 parties en poids d'au moins un catalyseur de polycondensation **A** tel que défini selon l'une quelconque des revendications 1 à 3.

6. Système bicomposant précurseur de la composition organopolysiloxanique telle que définie selon l'une quelconque des revendications précédentes et vulcanisable en élastomère silicone par des réactions de polycondensation et **caractérisé en ce qu'**il se présente en deux parties **P1** et **P2** distinctes destinées à être mélangées pour former ladite composition, et **en ce que** l'une de ces parties comprend le catalyseur de polycondensation **A** tel que défini selon l'une quelconque des revendications précédentes comme catalyseur de la réaction de polycondensation d'organopolysiloxanes et l'agent de réticulation **D** alors que l'autre partie est exempte des espèces précitées et comprend:
- pour 100 parties en poids de l'huile (ou des huiles) polyorganosiloxane(s) **C** susceptible(s) de réticuler par polycondensation en un élastomère, et
- de 0.001 à 10 partie(s) en poids d'eau.

7. Composition polyorganosiloxanique monocomposante stable au stockage en l'absence d'humidité et réticulant, en présence d'eau, en élastomère, **caractérisée en ce qu'**elle comprend:
- au moins une huile polyorganosiloxane **C** linéaire réticulable présentant des extrémités fonctionnalisées de type alcoxy, oxime, acyle et /ou énoxy, de préférence alcoxy,
- au moins un agent de réticulation **D,**
- au moins une charge **F,** et
- au moins un catalyseur de la réaction de polycondensation qui est le catalyseur de polycondensation **A** tel que défini selon l'une quelconque des revendications 1 à 3.

8. Composition polyorganosiloxanique monocomposante selon la revendication 7 **caractérisée en ce que** l'huile polyorganosiloxane **C** linéaire réticulable présentant des extrémités fonctionnalisées de type alcoxy est préparée in situ par réaction, en présence d'une quantité catalytiquement efficace de lithine, d'un diorganopolysiloxane linéaire, comprenant un groupement hydroxy lié à un atome de silicium à chaque extrémité de chaîne, avec au moins un polyalcoxysilane de formule **(II)** suivante:
(R⁴)_{c}(R⁵)ₐSi(OR⁶)_{4-(a+c)} **(II)**
dans laquelle:
- a est égal à 0, 1 ou 2,
- c est égal à 0, 1 ou 2,
- la somme a + c est égale à 0, 1 ou 2,
- R⁴ représente un radical monovalent hydrocarboné saturé ou non en C₁ à C₁₃, substitué ou non, aliphatique, cyclanique ou aromatique, R⁴ pouvant être identique à R⁵,
- R⁵ représente un radical monovalent hydrocarboné saturé ou non en C₁ à C_{13'}, substitué ou non substitué, aliphatique, cyclanique ou aromatique, et pouvant comporter une fonction époxy, amine primaire, secondaire, tertiaire, mercapto, et
- R⁶ représente un radical organique aliphatique ayant de 1 à 8 atomes de carbone choisi notamment entre les radicaux alkyle, les radicaux alkyléther, les radicaux alkylester, les radicaux alkylcétone, les radicaux alkylcyano et les radicaux aralkyle ayant de 7 à 13 atomes de carbone, étant entendu que les groupements alcoxy du silane de formule **(II)** peuvent avoir chacun une signification différente pour R⁶ ou la même signification.

9. Composition polyorganosiloxanique monocomposante selon la revendication 7 ou 8 caractérisée ce que l'agent de réticulation **D** ou le polyalcoxysilane de formule **(II)** sont choisis parmi le groupe constitué par:
- le vinyltriéthoxysilane,
- le méthyltriéthoxysilane,
- le propyltriéthoxysilane,
- le tétraéthoxysilane,
- le 1,2-bis(triéthoxysilyl)éthane,
- C₂H₅Si(OC₂H₅)₃, et
- Si(OC₂H₅)₄.

10. Elastomère obtenu par réticulation et durcissement du système bi-composant tel que défini selon la revendication 6 ou de la composition polyorganosiloxanique monocomposante telle que définie selon l'une quelconque de revendications 7 à 9 ou de la composition telle que définie selon l'une quelconque des revendications 1 à 5 .

11. Composés de formules:

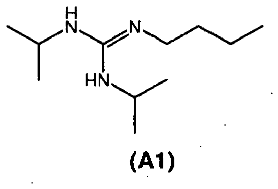

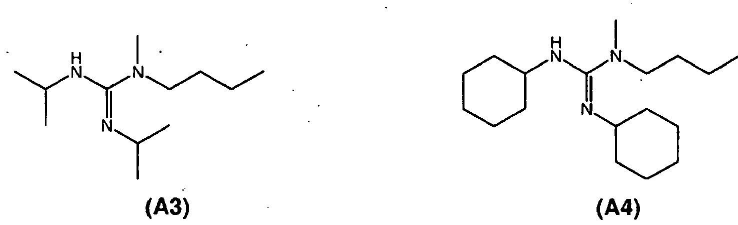

12. Utilisation pour catalyser.la réaction de polycondensation d'organopolysiloxanes d'un catalyseur de polycondensation **A** qui est un composé organique non silylé et répondant à la formule générale **(I):** 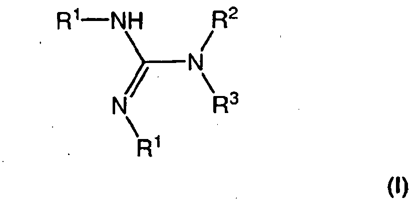 dans laquelle,
- les radicaux R¹, identiques ou différents, représentent, indépendamment l'un de l'autre, un groupe alkyle monovalent linéaire ou ramifié, un groupement cycloalkyle, un groupe (cycloalkyl)alkyle, le cycle étant substitué ou non et pouvant comprendre au moins un hétéroatome ou un groupement fluoroalkyle,
- le radical R² représente un atome d'hydrogène, un groupement alkyle monovalent linéaire ou ramifié, un groupement cycloalkyle, un groupement alkyle substitué par un cycle, substitué ou non et pouvant comprendre au moins un hétéroatome, un groupement aromatique un groupe arylalkyle, un groupement fluoroalkyle, un groupement alkylamine ou alkylguanidine, et
- le radical R³ représente un groupement alkyle monovalent linéaire ou ramifié, un groupement cycloalkyle, un groupement alkyle substitué par un cycle, substitué ou non et pouvant comprendre au moins un hétéroatome, un groupement arylalkyle, fluoroalkyle, alkylamine ou alkylguanidine,
- lorsque le radical R² n'est pas un atome d'hydrogène, les radicaux R² et R³ peuvent être liés pour former un cycle aliphatique à 3, 4, 5, 6 ou 7 chaînons éventuellement substitué par un ou plusieurs substituants, et
- avec la condition supplémentaire que les radicaux R¹, R² et R³ ne comprennent pas d'atome de silicium.

13. Utilisation pour catalyser la réaction de polycondensation d'organopolysiloxanes d'un catalyseur de polycondensation **A** qui est un composé organique non silylé choisi parmi le groupe constitué par les composés **(A1)** à **(A6)** suivants:

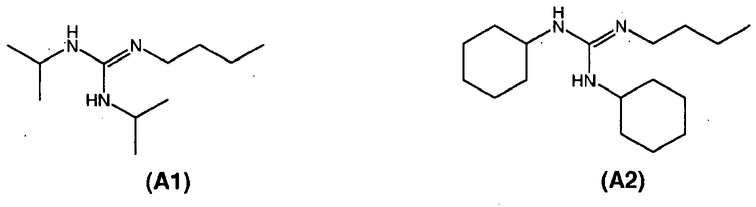

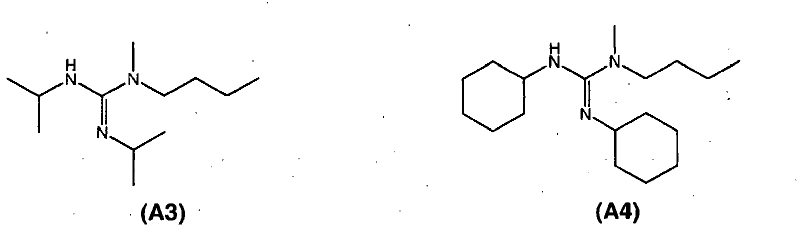

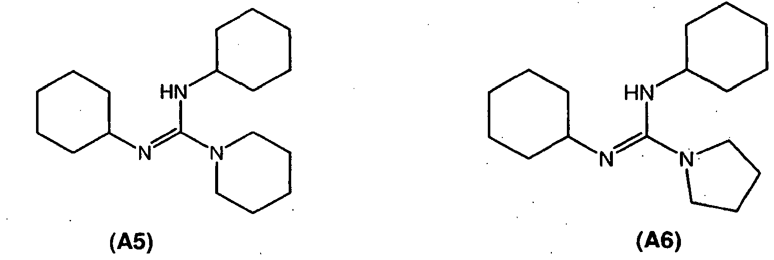

## Patentansprüche

1. Organopolysiloxanzusammensetzung, die keinen Metallkatalysator enthält und **dadurch gekennzeichnet ist, dass** sie einerseits eine Silikonbasis **B,** die mindestens ein Polyorganosiloxanöl umfasst, das durch Polykondensationsreaktion zu einem Silikonelastomer vernetzt werden kann, und andererseits eine katalytisch wirksame Menge mindestens eines Polykondensationskatalysators **A,** bei dem es sich um eine nichtsilylierte organische Verbindung der allgemeinen Formel **(I)** handelt: 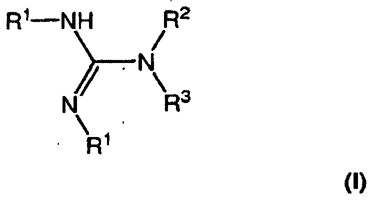 worin
- die Reste R¹, die gleich oder verschieden sind, unabhängig voneinander für eine lineare oder verzweigte einwertige Alkylgruppe, eine Cycloalkylgruppe, eine (Cycloalkyl)alkylgruppe, wobei der Ring substituiert oder unsubstituiert ist und mindestens ein Heteroatom enthalten kann, oder eine Fluoralkylgruppe stehen,
- der Rest R² für ein Wasserstoffatom, eine lineare oder verzweigte einwertige Alkylgruppe, eine Cycloalkylgruppe, eine durch einen Ring substituierte Alkylgruppe, wobei der Ring substituiert oder unsubstituiert ist und mindestens ein Heteroatom enthalten kann, eine aromatische Gruppe, eine Arylalkylgruppe, eine Fluoralkylgruppe, eine Alkylamingruppe oder eine Alkylguanidingruppe steht und
- der Rest R³ für eine lineare oder verzweigte einwertige Alkylgruppe, eine Cycloalkylgruppe, eine durch einen Ring substituierte Alkylgruppe, wobei der Ring substituiert oder unsubstituiert ist und mindestens ein Heteroatom enthalten kann, eine Arylalkylgruppe, eine Fluoralkylgruppe, eine Alkylamingruppe oder eine Alkylguanidingruppe steht,
- dann, wenn der Rest R² nicht für ein Wasserstoffatom steht, die Reste R² und R³ unter Bildung eines 3-, 4-, 5-, 6- oder 7-gliedrigen aliphatischen Rings, der gegebenenfalls durch einen oder mehrere Substituenten substituiert ist, verbunden sein können, und
- mit der zusätzlichen Maßgabe, dass die Reste R¹, R² und R³ kein Siliciumatom enthalten,
umfasst.

2. Organopolysiloxanzusammensetzung nach Anspruch 1, **dadurch gekennzeichnet, dass** es sich bei dem Polykondensationskatalysator **A** um eine nichtsilylierte organische Verbindung der allgemeinen Formel **(I)** handelt und worin:
- die Reste R¹, die gleich oder verschieden sind, und der Rest R³ unabhängig voneinander aus der Gruppe bestehend aus einem Isopropylrest, einem Cyclohexylrest und einem linearen oder verzweigten einwertigen C₁-C₁₂-Alkylrest ausgewählt sind,
- der Rest R² für ein Wasserstoffatom, eine lineare oder verzweigte einwertige Alkylgruppe, eine Cycloalkylgruppe, eine durch einen Ring substituierte Alkylgruppe, wobei der Ring substituiert oder unsubstituiert ist und mindestens ein Heteroatom enthalten kann, eine Arylalkylgruppe, eine Fluoralkylgruppe, eine Alkylamingruppe oder eine Alkylguanidingruppe steht und
- dann, wenn der Rest R² nicht für ein Wasserstoffatom steht, die Reste R² und R³ unter Bildung eines 3-, 4-, 5-, 6- oder 7-gliedrigen aliphatischen Rings, der gegebenenfalls durch einen oder mehrere Substituenten substituiert ist, verbunden sein können.

3. Organopolysiloxanzusammensetzung nach Anspruch 2, wobei es sich bei dem Polykondensationskatalysator **A** um eine nichtsilylierte organische Verbindung handelt, die aus den folgenden Verbindungen (**A1**) bis (**A6**) ausgewählt ist:

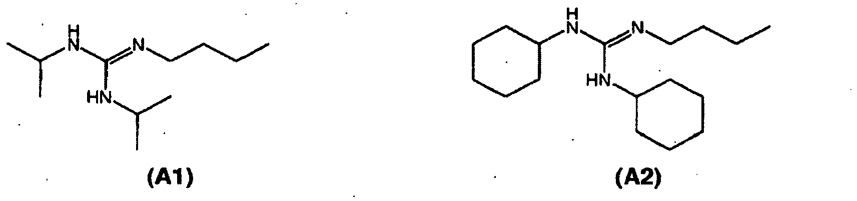

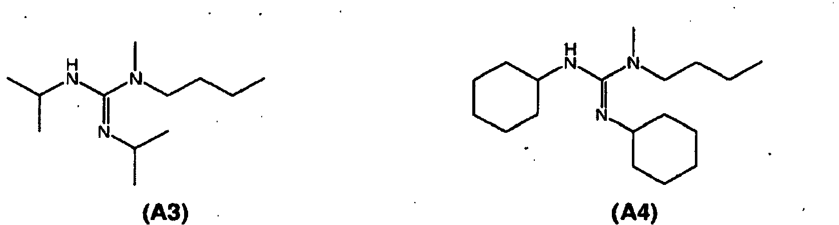

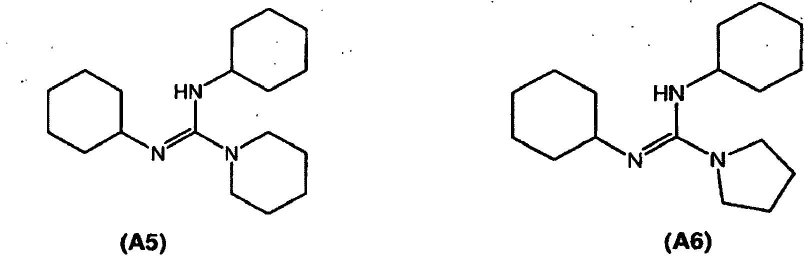

4. Zusammensetzung nach einem der vorhergehenden Ansprüche, **dadurch gekennzeichnet, dass** sie außerdem eine katalytisch wirksame Menge mindestens eines Polykondensationskatalysators **A** gemäß der in einem der Ansprüche 1 bis 3 angegebenen Definition und eine Silikonbasis **B,** umfassend:
- mindestens ein Polyorganosiloxanöl **C,** das durch Polykondensation zu einem Elastomer vernetzt werden kann;
- gegebenenfalls mindestens ein Vernetzungsmittel **D**;
- gegebenenfalls mindestens einen Haftvermittler **E**; und
- gegebenenfalls mindestens einen siliciumhaltigen, organischen und/oder nicht siliciumhaltigen mineralischen Füllstoff **F**;
umfasst.

5. Organopolysiloxanzusammensetzung nach Anspruch 4, die durch Polykondensationsreaktionen zu einem Silikonelastomer vulkanisierbar ist, wobei:
(a) die Silikonbasis **B:**
- auf 100 Gewichtsteile mindestens eines durch Polykondensation vernetzbaren Polyorganosiloxanöls **C,** bei dem es sich um ein reaktives α,ω-Dihydroxydiorganopolysiloxanpolymer handelt, bei dessen organischen Resten es sich um Kohlenwasserstoffreste handelt, die vorzugsweise aus der Gruppe bestehend aus Alkylgruppen mit 1 bis 20 Kohlenstoffatomen; Cycloalkylgruppen mit 3 bis 8 Kohlenstoffatomen; Alkenylgruppen mit 2 bis 8 Kohlenstoffatomen und Cycloalkenylgruppen mit 5 bis 8 Kohlenstoffatomen ausgewählt sind;
- 0,1 bis 60 Gewichtsteile mindestens eines Vernetzungsmittels **D,** das aus der Gruppe bestehend aus Polyalkoxysilanen, Produkten der Partialhydrolyse eines Polyalkoxysilans und Polyalkoxysiloxanen ausgewählt ist;
- 0 bis 60 Gewichtsteile eines Haftvermittlers **E** gemäß obiger Beschreibung;
- 0 bis 250 Gewichtsteile, vorzugsweise 5 bis 200 Gewichtsteile, mindestens eines siliciumhaltigen, organischen und/oder nicht siliciumhaltigen mineralischen Füllstoffs **F**;
- 0,001 bis 10 Gewichtsteile Wasser,
- 0 bis 100 Gewichtsteile mindestens eines nichtreaktiven linearen Polyorganosiloxanpolymers **G**, das aus einem linearen Homopolymer oder Copolymer besteht, dessen einwertige organische Substituenten, die gleich oder voneinander verschieden sind und an die Siliciumatome gebunden sind, pro Molekül aus Alkyl-, Cycloalkyl-, Alkenyl-, Aryl-, Alkylarylen- und Arylalkylenresten ausgewählt sind,
- 0 bis 20 Gewichtsteile einer Farbbasis oder eines Farbmittels **H**,
- 0 bis 70 Gewichtsteile Polyorganosiloxanharze **I** und
- 0 bis 20 Teile Hilfsadditive **J**, die dem Fachmann bekannt sind, wie Weichmacher, Vernetzungsverzögerer, Mineralöle, antimikrobielle Mittel, Wärmebeständigkeitsadditive wie Titan-, Eisen- oder Ceroxide, und
(b) 0,1 bis 50 Gewichtsteile mindestens eines Polykondensationskatalysators **A** gemäß einem der Ansprüche 1 bis 3
umfasst.

6. Zweikomponentensystem, das einen Vorläufer für die Organopolysiloxanzusammensetzung gemäß einem der vorhergehenden Ansprüche darstellt und durch Polykondensationsreaktionen zu einem Silikonelastomer vulkanisierbar ist und **dadurch gekennzeichnet ist, dass** es zwei getrennte Teile **P1** und **P2,** die zum Mischen zur Bildung der Zusammensetzung vorgesehen sind, aufweist und einer dieser Teile den Polykondensationskatalysator **A** gemäß einem der vorhergehenden Ansprüche als Katalysator für Polykondensationsreaktionen von Organopolysiloxanen und das Vernetzungsmittel **D** umfasst, wohingegen der andere Teil von den oben angegebenen Spezies frei ist, und
- auf 100 Gewichtsteile des Polyorganosiloxanöls bzw. der Polyorganosiloxanöle **C,** das bzw. die durch Polykondensation zu einem Elastomer vernetzt werden kann bzw. können, und
- 0,001 bis 10 Gewichtsteile Wasser umfasst.

7. Einkomponentige Polyorganosiloxanzusammensetzung, die in Abwesenheit von Feuchtigkeit lagerstabil ist und in Gegenwart von Wasser zu einem Elastomer vernetzt, **dadurch gekennzeichnet, dass** sie:
- mindestens ein vernetzbares lineares Polyorganosiloxanöl **C,** das funktionalisierte Enden vom Alkoxy-, Oxim-, Acyl- und/oder Enoxy-Typ, vorzugsweise vom Alkoxy-Typ, aufweist,
- mindestens ein Vernetzungsmittel **D,**
- mindestens einen Füllstoff **F** und
- mindestens einen Katalysator für die Polykondensationsreaktion, bei dem es sich um den Polykondensationskatalysator **A** gemäß einem der Ansprüche 1 bis 3 handelt,
umfasst.

8. Einkomponentige Polyorganosiloxanzusammensetzung nach Anspruch 7, **dadurch gekennzeichnet, dass** das vernetzbare lineare Polyorganosiloxanöl **C,** das funktionalisierte Enden vom Alkoxy-Typ aufweist, in situ durch Umsetzung eines linearen Diorganopolysiloxans, das an jedem Kettenende eine an ein Siliciumatom gebundene Hydroxylgruppe aufweist, mit mindestens einem Polyalkoxysilan der folgenden Formel **(II):**
(R⁴)_{c}(R⁵)ₐSi(OR⁶)_{4-(a+c)} **(II)**
worin:
- a gleich 0, 1 oder 2 ist,
- c gleich 0, 1 oder 2 ist,
- die Summe a + c gleich 0, 1 oder 2 ist,
- R⁴ für einen aliphatischen, cyclanischen oder aromatischen, substituierten oder unsubstituierten, gesättigten oder ungesättigten einwertigen C₁-bis C₁₃-Kohlenwasserstoffrest steht, wobei R⁴ mit R⁵ identisch sein kann,
- R⁵ für einen aliphatischen, cyclanischen oder aromatischen, substituierten oder unsubstituierten, gesättigten oder ungesättigten einwertigen C₁-bis C₁₃-Kohlenwasserstoffrest, der eine Epoxid-, primäre, sekundäre oder tertiäre Amin- oder Mercaptofunktion enthalten kann, steht, und
- R⁶ für einen aliphatischen organischen Rest mit 1 bis 8 Kohlenstoffatomen, der insbesondere aus Alkylresten, Alkyletherresten, Alkylesterresten, Alkylketonresten, Alkylcyanoresten und Aralkylresten mit 7 bis 13 Kohlenstoffatomen ausgewählt ist, steht, mit der Maßgabe, dass die Alkoxygruppen des Silans der Formel **(II)** jeweils eine andere Bedeutung für R⁶ oder die gleiche Bedeutung haben können,
in Gegenwart einer katalytisch wirksamen Menge von Lithiumhydroxid hergestellt wird.

9. Einkomponentige Polyorganosiloxanzusammensetzung nach Anspruch 7 oder 8, **dadurch gekennzeichnet, dass** das Vernetzungsmittel **D** oder das Polyalkoxysilan der Formel **(II)** aus der Gruppe bestehend aus:
- Vinyltriethoxysilan,
- Methyltriethoxysilan,
- Propyltriethoxysilan,
- Tetraethoxysilan,
- 1,2-Bis(triethoxysilyl)ethan,
- C₂H₅Si(OC₂H₅)₃ und
- Si(OC₂H₅)₄
ausgewählt sind.

10. Elastomer, erhalten durch Vernetzung und Härtung des Zweikomponentensystems gemäß Anspruch 6 oder der einkomponentigen Polyorganosiloxanzusammensetzung gemäß einem der Ansprüche 7 bis 9 oder der Zusammensetzung gemäß einem der Ansprüche 1 bis 5.

11. Verbindungen der Formel:

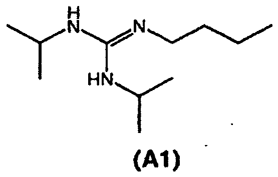

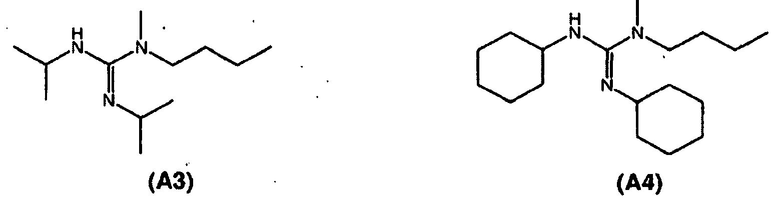

12. Verwendung eines Polykondensationskatalysators **A,** bei dem es sich um eine nichtsilylierte organische Verbindung der allgemeinen Formel **(I)** handelt: 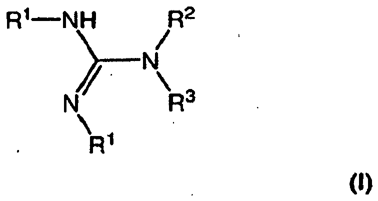 worin
- die Reste R¹, die gleich oder verschieden sind, unabhängig voneinander für eine lineare oder verzweigte einwertige Alkylgruppe, eine Cycloalkylgruppe, eine (Cycloalkyl)alkylgruppe, wobei der Ring substituiert oder unsubstituiert ist und mindestens ein Heteroatom enthalten kann, oder eine Fluoralkylgruppe stehen,
- der Rest R² für ein Wasserstoffatom, eine lineare oder verzweigte einwertige Alkylgruppe, eine Cycloalkylgruppe, eine durch einen Ring substituierte Alkylgruppe, wobei der Ring substituiert oder unsubstituiert ist und mindestens ein Heteroatom enthalten kann, eine aromatische Gruppe, eine Arylalkylgruppe, eine Fluoralkylgruppe, eine Alkylamingruppe oder eine Alkylguanidingruppe steht und
- der Rest R³ für eine lineare oder verzweigte einwertige Alkylgruppe, eine Cycloalkylgruppe, eine durch einen Ring substituierte Alkylgruppe, wobei der Ring substituiert oder unsubstituiert ist und mindestens ein Heteroatom enthalten kann, eine Arylalkylgruppe, eine Fluoralkylgruppe, eine Alkylamingruppe oder eine Alkylguanidingruppe steht,
- dann, wenn der Rest R² nicht für ein Wasserstoffatom steht, die Reste R² und R³ unter Bildung eines 3-, 4-, 5-, 6- oder 7-gliedrigen aliphatischen Rings, der gegebenenfalls durch einen oder mehrere Substituenten substituiert ist, verbunden sein können, und
- mit der zusätzlichen Maßgabe, dass die Reste R¹, R² und R³ kein Siliciumatom enthalten,
zur Katalyse der Polykondensationsreaktion von Organopolysiloxanen.

13. Verwendung eines Polykondensationskatalysators **A,** bei dem es sich um eine nichtsilylierte organische Verbindung handelt, die aus den folgenden Verbindungen (**A1**) bis (**A6**) ausgewählt ist: 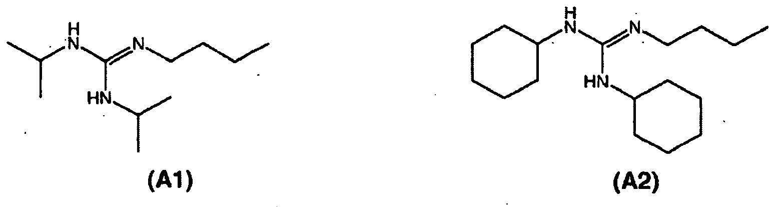 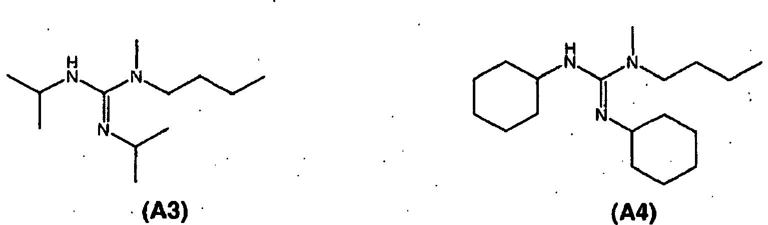 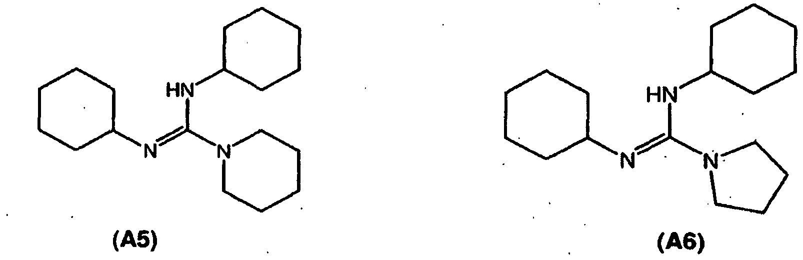 zur Katalyse der Polykondensationsreaktion von Organopolysiloxanen.

## Claims

1. Organopolysiloxane composition which does not contain any metal catalyst and which is **characterized in that** it comprises, firstly, a silicone base **B** comprising at least one polyorganosiloxane oil which can be crosslinked via a polycondensation reaction so as to form a silicone elastomer and, secondly, a catalytically effective amount of at least one polycondensation catalyst **A** which is a nonsilylated organic compound corresponding to general formula **(I):** 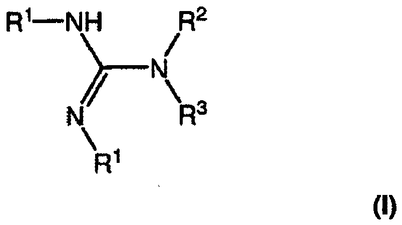 in which:
- the R¹ radicals, which may be identical or different, represent, independently of one another, a linear or branched monovalent alkyl group, a cycloalkyl group, a (cycloalkyl)alkyl group, the ring being substituted or unsubstituted and being able to comprise at least one heteroatom, or a fluoroalkyl group,
- the R² radical represents a hydrogen atom, a linear or branched monovalent alkyl group, a cycloalkyl group, an alkyl group substituted with a ring, which is substituted or unsubstituted and which can comprise at least one heteroatom, an aromatic group, an arylalkyl group, a fluoroalkyl group, an alkylamine group or an alkylguanidine group, and
- the R³ radical represents a linear or branched monovalent alkyl group, a cycloalkyl group, an alkyl group substituted with a ring, which is substituted or unsubstituted and which can comprise at least one heteroatom, an arylalkyl group, a fluoroalkyl group, an alkylamine group or an alkylguanidine group,
- when the R² radical is not a hydrogen atom, the R² and R³ radicals can be linked so as to form a 3-, 4-, 5-, 6- or 7-membered aliphatic ring optionally substituted with one or more substituents, and
- with the additional condition that the R¹, R² and R³ radicals do not comprise a silicon atom.

2. Organopolysiloxane composition according to Claim 1, **characterized in that** the polycondensation catalyst **A** is a nonsilylated organic compound corresponding to general formula **(I)** and in which:
- the R₁ radicals, which may be identical or different, and the R₃ radical are chosen, independently of one another, from the group consisting of: an isopropyl radical, a cyclohexyl radical and a linear or branched, monovalent C₁-C₁₂ alkyl radical,
- the R₂ radical represents a hydrogen atom, a linear or branched monovalent alkyl group, a cycloalkyl group, an alkyl group substituted with a ring, which is substituted or unsubstituted and which can comprise at least one heteroatom, an arylalkyl group, a fluoroalkyl group, an alkylamine group or an alkylguanidine group, and
- when the R² radical is not a hydrogen atom, the R² and R³ radicals can be linked so as to form a 3-, 4-, 5-, 6- or 7-membered aliphatic ring optionally substituted with one or more substituents.

3. Organopolysiloxane composition according to Claim 2, in which the polycondensation catalyst **A** is a nonsilylated organic compound chosen from the group constituted of the following compounds **(A1)** to **(A6):**

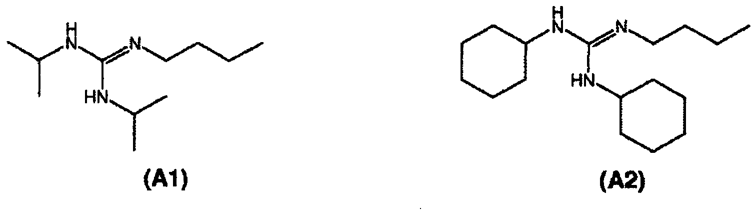

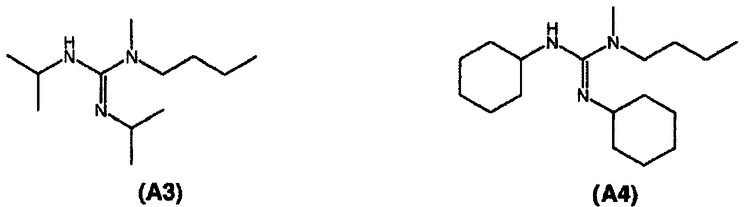

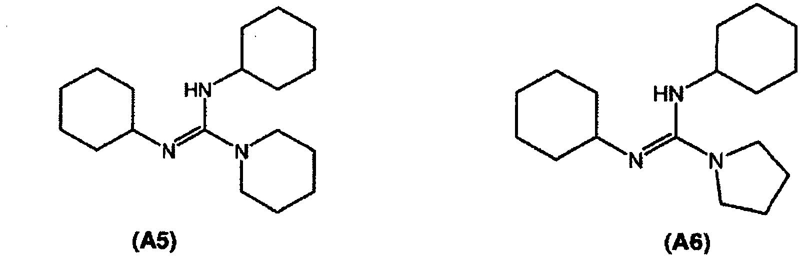

4. Composition according to any one of the preceding claims, **characterized in that** it also comprises a catalytically effective amount of at least one polycondensation catalyst **A** as defined in any one of Claims 1 to 3 and a silicone base **B** comprising:
- at least one polyorganosiloxane oil **C** capable of crosslinking by polycondensation to give an elastomer;
- optionally, at least one crosslinking agent **D;**
- optionally, at least one adhesion promoter **E;** and
- optionally, at least one siliceous, organic and/or nonsiliceous mineral filler **F.**

5. Organopolysiloxane composition according to Claim 4 which can be vulcanized to give a silicone elastomer via polycondensation reactions, in which:
(a) the silicone base **B** comprises:
- for 100 parts by weight of at least one polyorganosiloxane oil **C** capable of crosslinking by polycondensation, which is an α,ω-dihydroxydiorganopolysiloxane reactive polymer of which the organic radicals are hydrocarbon-based radicals, preferably chosen from the group consisting of: alkyls containing from 1 to 20 carbon atoms; cycloalkyls containing from 3 to 8 carbon atoms; alkenyls containing from 2 to 8 carbon atoms and cycloalkenyls containing from 5 to 8 carbon atoms,
- from 0.1 to 60 parts by weight of at least one crosslinking agent **D** chosen from the group constituted of: polyalkoxysilanes, products originating from the partial hydrolysis of a polyalkoxysilane, and polyalkoxysiloxanes,
- from 0 to 60 parts by weight of an adhesion promoter **E** as described above,
- from 0 to 250 parts by weight, preferably from 5 to 200 parts by weight, of at least one siliceous, organic and/or nonsiliceous mineral filler **F,**
- from 0.001 to 10 parts by weight of water,
- from 0 to 100 parts by weight of at least one nonreactive, linear polyorganosiloxane polymer **G** consisting of a linear homopolymer or copolymer of which, per molecule, the monovalent organic substituents, which may be identical to or different from one another, and which are bonded to the silicon atoms, are chosen from alkyl, cycloalkyl, alkenyl, aryl, alkylarylene and arylalkylene radicals,
- from 0 to 20 parts by weight of a colouring base or of a colouring agent **H,**
- from 0 to 70 parts by weight of polyorganosiloxane resins **I,** and
- from 0 to 20 parts of auxiliary additives **J** known to those skilled in the art, such as plasticizers, crosslinking retardants, mineral oils, antimicrobial agents or heat stabilizers, such as titanium, iron or cerium oxides, and
(b) from 0.1 to 50 parts by weight of at least one polycondensation catalyst **A** as defined in any one of Claims 1 to 3.

6. Two-component system which is a precursor of the organopolysiloxane composition as defined in any one of the preceding claims and which can be vulcanized to give a silicone elastomer via polycondensation reactions and which is **characterized in that** it is in two separate parts **P1** and **P2** intended to be mixed so as to form said composition, and **in that** one of these parts comprises the polycondensation catalyst **A** as defined in any one of the preceding claims as catalyst of the reaction for polycondensation of organopolysiloxanes and the crosslinking agent **D,** whereas the other part is free of the abovementioned species and comprises:
- for 100 parts by weight of the polyorganosiloxane oil(s) **C** capable of crosslinking by polycondensation to give an elastomer, and
- from 0.001 to 10 part(s) by weight of water.

7. Single-component polyorganosiloxane composition which is stable during storage in the absence of moisture and which crosslinks, in the presence of water, to give an elastomer, **characterized in that** it comprises:
- at least one crosslinkable linear polyorganosiloxane oil **C** which has functionalized ends of alkoxy, oxime, acyl and/or enoxy type, preferably alkoxy type,
- at least one crosslinking agent **D,**
- at least one filler **F,** and
- at least one catalyst of the polycondensation reaction which is the polycondensation catalyst **A** as defined in any one of Claims 1 to 3.

8. Single-component polyorganosiloxane composition according to Claim 7, **characterized in that** the crosslinkable linear polyorganosiloxane oil C which has functionalized ends of alkoxy type is prepared in situ by reaction, in the presence of a catalytically effective amount of lithium hydroxide, of a linear diorganopolysiloxane comprising a hydroxyl group bonded to a silicon atom at each chain end, with at least one polyalkoxysilane of the formula **(II)** below:
(R⁴)_{c}(R⁵)ₐSi(OR⁶)_{4-(a+c)} **(II)**
in which:
- a is equal to 0, 1 or 2,
- c is equal to 0, 1 or 2,
- the sum of a+c is equal to 0, 1 or 2,
- R⁴ represents an aliphatic, cyclanic or aromatic, substituted or unsubstituted, and saturated or unsaturated C₁ to C₁₃ hydrocarbon-based monovalent radical, it being possible for R⁴ to be identical to R⁵,
- R⁵ represents an aliphatic, cyclanic or aromatic, substituted or unsubstituted, and saturated or unsaturated C₁ to C₁₃ hydrocarbon-based monovalent radical which can comprise an epoxy, primary, secondary or tertiary amine, or mercapto function, and
- R⁶ represents an aliphatic organic radical containing from 1 to 8 carbon atoms, chosen in particular from alkyl radicals, alkyl ether radicals, alkyl ester radicals, alkyl ketone radicals, alkylcyano radicals and aralkyl radicals containing from 7 to 13 carbon atoms, it being understood that the alkoxy groups of the silane of formula **(II)** can each have a different meaning for R⁶ or the same meaning.

9. Single-component polyorganosiloxane composition according to Claim 7 or 8, **characterized in that** the crosslinking agent **D** or the polyalkoxysilane of formula **(II)** are chosen from the group constituted of:
- vinyltriethoxysilane,
- methyltriethoxysilane,
- propyltriethoxysilane,
- tetraethoxysilane,
- 1,2-bis(triethoxysilyl)ethane,
- C₂H₅Si(OC₂H₅)₃, and
- Si(OC₂H₅)₄.

10. Elastomer obtained by crosslinking and curing of the two-component system as defined in Claim 6 or of the single-component polyorganosiloxane composition as defined in any one of Claims 7 to 9 or of the composition as defined in any one of Claims 1 to 5.

11. Compounds of formulae:

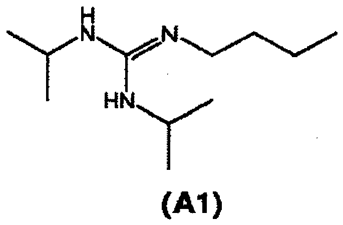

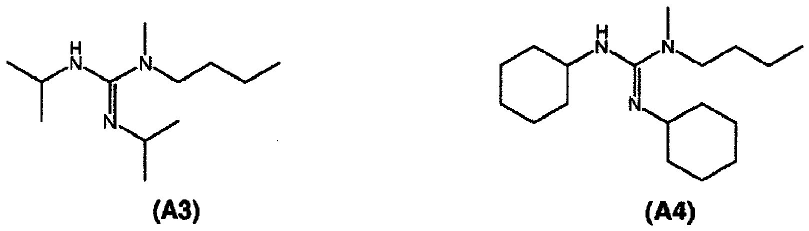

12. Use, for catalyzing the organopolysiloxane polycondensation reaction, of a polycondensation catalyst **A** which is a nonsilylated organic compound corresponding to general formula **(I)**: 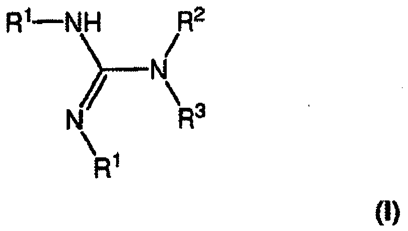 in which:
- the R¹ radicals, which may be identical or different, represent, independently of one another, a linear or branched monovalent alkyl group, a cycloalkyl group, a (cycloalkyl)alkyl group, the ring being substituted or unsubstituted and being able to comprise at least one heteroatom, or a fluoroalkyl group,
- the R² radical represents a hydrogen atom, a linear or branched monovalent alkyl group, a cycloalkyl group, an alkyl group substituted with a ring, which is substituted or unsubstituted and which can comprise at least one heteroatom, an aromatic group, an arylalkyl group, a fluoroalkyl group, an alkylamine group or an alkylguanidine group, and
- the R³ radical represents a linear or branched monovalent alkyl group, a cycloalkyl group, an alkyl group substituted with a ring, which is substituted or unsubstituted and which can comprise at least one heteroatom, an arylalkyl group, a fluoroalkyl group, an alkylamine group or an alkylguanidine group,
- when the R² radical is not a hydrogen atom, the R² and R³ radicals can be linked so as to form a 3-, 4-, 5-, 6- or 7-membered aliphatic ring optionally substituted with one or more substituents, and
- with the additional proviso that the R¹, R² and R³ radicals do not comprise a silicon atom.

13. Use, for catalyzing the organopolysiloxane polycondensation reaction, of a polycondensation catalyst **A** which is a nonsilylated organic compound chosen from the group constituted of the following compounds **(A1)** to **(A6)**:
